# EUROPEAN PATENT APPLICATION

(11) **EP 2 630 968 A1**
(43) Date of publication of application: **28.08.2013**
(21) Application number: 13159462.4
(22) Date of filing: 29.06.2007
(51) Int. Cl.: A61K 39/00, A61K 39/395, C12N 5/0784

(54) **Dendritic cells generated using GM-CSF and interferon alpha and loaded with heat-treated and killed cancer cells**

(30) Priority: 30.06.2006 US 817916 P
(62) Divisional of application: 07812497.1
(71) Applicant: Baylor Research Institute, Dallas, TX 75204 (US)
(72) Inventor: Palucka, Anna Karolina, Dallas, TX 75204 (US); Banchereau, Jacques F., Dallas, TX 75230 (US); Taquet, Nicolas, Dallas, TX 75248 (US); Burkeholder, Susan, Fort Worth, TX 76118 (US)
(74) Representative: Sonn & Partner Patentanwälte

(57) **Abstract**

The present invention includes compositions and methods for making a pharmaceutically effective amount of one or more dendritic cells matured by exposure to IFNα loaded with cancer antigens from cells that are heat shocked and subsequently killed, wherein the one or more IFNα dendritic cells are under conditions to present the one or more cancer antigens are presented within the context of autolo

## Description

### TECHNICAL FIELD OF THE INVENTION

The present invention relates in general to the field of vaccines and, more particularly, to compositions and methods for the production of dendritic cells generated using dendritic cell activation factors and loaded with heat-treated and killed cancer cells.

### BACKGROUND OF THE INVENTION

Without limiting the scope of the invention, its background is described in connection with cancer cell vaccines.

Numerous methods for the isolation, characterization and induction of immune responses have been developed. For example, in United States Patent No. 6,821,778, issued to Engleman, et al., certain methods for using dendritic cells to activate gammaldelta-T cell receptor-positive T cells are taught. Briefly, methods of using human dendritic cells to present antigens for the induction of antigen-specific T cell-mediated immune responses are taught. In particular, the disclosure includes the isolation of dendritic cells from human blood, exposing the cells to antigens, co-culturing the antigen-pulsed dendritic cells with gamma/delta-T cell receptor-positive-T cells (gamma/delta-TCR+ T cells) obtained from unprimed or weakly primed individuals for the stimulation of antigen-specific T cell proliferative and cytotoxic activities. The dendritic cell antigen presentation system described therein is said to have wide range of applications, e.g., activation and expansion of large numbers of antigen-specific major histocompatibility complex-unrestricted T cells for use in adoptive cellular immunotherapy against infectious diseases and cancer.

Yet another example of dendritic cell manipulation is taught in United States Patent No. 6,652,848, issued to Gong, et al., which teaches dendritic cell hybrids. Gong teaches immunostimulatory compositions that contain fused cells formed by fusion between dendritic cells and non-dendritic cells, methods of using these compositions, and methods of generating dendritic cell hybrids.

United States Patent No. 6,602,709, issued to Albert, et al., includes methods for use of apoptotic cells to deliver antigen to dendritic cells for induction or tolerization of T cells. The disclosure teaches methods and compositions useful for delivering antigens to dendritic cells which are then useful for inducing antigen-specific cytotoxic T lymphocytes and T helper cells. The disclosure is also said to provide assays for evaluating the activity of cytotoxic T lymphocytes. Briefly, antigens are targeted to dendritic cells by apoptotic cells, which may be modified to express non-native antigens for presentation to the dendritic cells. The dendritic cells are primed by the apoptotic cells are said to be capable of processing and presenting the processed antigen and inducing cytotoxic T lymphocyte activity or may also be used in vaccine therapies.

United States Patent No. 6,475,483, issued to Steinman, et al., disclosed methods for in vitro proliferation of dendritic cell precursors and their use to produce immunogens for treating autoimmune diseases. The disclosure is said to teach methods for producing proliferating cultures of dendritic cell precursors is provided, as well as methods for producing mature dendritic cells in culture from the proliferating dendritic cell precursors. The cultures of mature dendritic cells provide an effective means of producing novel T cell dependent antigens that consist of dendritic cell modified antigens or dendritic cells pulsed with antigen, including particulates, which antigen is processed and expressed on the antigen-activated dendritic cell. The novel antigens of the invention may be used as immunogens for vaccines or for the treatment of disease. These antigens may also be used to treat autoimmune diseases such as juvenile diabetes and multiple sclerosis.

### SUMMARY OF THE INVENTION

The present invention includes compositions and methods for inducing immune responses that are coordinated and regulated by dendritic cells (DCs)^{1,2}. DCs are present in peripheral tissues, where they are poised to capture antigens. These antigens are subsequently processed into small peptides as the DCs mature and move towards the draining secondary lymphoid organs³. There, the DCs present the peptides to naïve T cells, thus inducing a cellular immune response, involving both T helper 1 (Th1) type CD4⁺ T cells and cytolytic CD8⁺ T cells. DCs are important in launching humoral immunity through their capacity to activate naïve⁴ and memory⁵ B cells. DCs can also activate natural killer (NK) cells ⁶ and NKT cells⁷. Because of their many capabilities, DCs are able to conduct all elements of the immune orchestra and therefore represent a fundamental target and tool for vaccination.

Ex vivo-generated and antigen-loaded DCs have recently been used as vaccines to improve immunity⁸. Numerous studies in mice have shown that DCs loaded with tumor antigens can induce therapeutic and protective anti-tumor immunity⁹. The immunogenicity of antigens delivered on DCs has been shown in patients with cancer⁸ and chronic HIV infection¹⁰, thus providing a "proof-of-principle" that DC vaccines can be effective. The identification of distinct DC subsets that induce distinct types of immune response and the role of DCs in the expansion of cells with regulatory/suppressor function provide novel parameters to be tested for design of better vaccination strategies for patients with cancer.

The present invention includes compositions and methods for the activation and presentation of antigen by antigen presenting cells. More particularly, the present invention include a method of making a composition for treating cancer by activating one or more dendritic cells that present one or more cancer antigens and induce T cell activation through incubation of the one or more dendritic cells with one or more cancer cells that are heat shocked and subsequently killed. The method may also include the step of pulsing the antigen presenting cells with an antigen comprising the remains of one or more cancer cells that are heat shocked and subsequently killed under conditions that induce T-cell activation. The method may also include the step of storing cryogenically the one or more antigen presenting cells that have been pulsed with the cancer cells that are heat shocked and subsequently killed. The one or more antigen presenting cells may be dendritic cells that present one or more cancer specific antigen obtained after heat shock and killing of the cancer cells to one or more CD8+ or CD4+ T cells. The antigen presenting cells may be dendritic cells, monocytes, autologous cells, heterologous cells, cell fragments and/or a combination thereof.

In certain examples, the one or more antigen presenting dendritic cells include one or more dendritic cells, e.g., monocytes that have been activated with IFNα. The antigen presenting cells may be derived from monocytes cultured with GM-CSF and IFNα. Examples of antigen presenting cells may be antigen presenting dendritic cells that are expressing one or more melanoma cell-derived heat shock proteins, e.g., heat inactivated melanoma cells derived from a patient. Alternatively, the one or more heat inactivated melanoma cells may be of the same basic cell type or even cancer cell type as the patient. In certain embodiments, the cancer cells are Colo829 melanoma cells. In one example, the cancer cells are established cancer cell lines, e.g., cultured melanoma cells, e.g., Mel-2, Mel-3, Mel-4, Mel-6 and/or Mel-9 cells or a combination thereof.

In certain embodiments, the cancer cells are treated by heating for about 0.5 to 4 hours at between about 38°C and about 46°C. The cancer cells may be killed by gamma-irradiation for about 0.5 hours at about 160Gy and/or killed by the heating, freeze-thaw cycles, French press, shearing, direct or indirect compression, freezing, cracking, drying, chemical exposure and biological agents that cause cell death (other than apoptosis) and the like. The present invention also includes the addition of one or more pulsed antigen presenting cells, which may include, enucleated dendritic cells and one or more heat treated cancer cells capable of inducing T cell activation, pulsed with a heat-shocked cells that is subsequently killed and processed for presentation. In certain embodiments, the composition is injected subcutaneously in at least one site selected from the group consisting of an anterior thigh, or an upper arm.

Non-limiting examples of cancer cells for heat-tretment and killing to make an antigen for presentation may include cells derived from, e.g., fibrosarcoma, myxosarcoma, liposarcoma, chondrosarcoma, osteogenic sarcoma, chordoma, angiosarcoma, Kaposi's sarcoma, endotheliosarcoma, lymphangiosarcoma, lymphangioendotheliosarcoma, synovioma, mesothelioma, Ewing's tumor, leiomyosarcoma, rhabdomyosarcoma, rhabdosarcoma, colorectal carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, neuroblastoma, retinoblastoma, myeloma, lymphoma, and leukemia.

The cancer that may be used to pulse the cells and/or that is the subject of treatment may include fibrosarcoma, myxosarcoma, liposarcoma, chondrosarcoma, osteogenic sarcoma, chordoma, angiosarcoma, Kaposi's sarcoma, endotheliosarcoma, lymphangiosarcoma, lymphangioendotheliosarcoma, synovioma, mesothelioma, Ewing's tumor, leiomyosarcoma, rhabdomyosarcoma, rhabdosarcoma, colorectal carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, neuroblastoma, retinoblastoma, myeloma, lymphoma, and leukemia.

Yet another embodiment is a method of making a dendritic cell that presents cancer antigens by differentiating one or more monocytes into one or more dendritic cells and loading the one or more dendritic cells with an antigen presenting composition that includes one or more cancer cells that are heat shocked and subsequently killed, wherein the one or more dendritic cells are under conditions that cause the one or more dendritic cells to present one or more antigens in the composition.

Another method of the present invention is a method of making an IFNα dendritic cell that presents melonoma antigens by isolating one or more monocytes from a patient suspected of having cancer; maturing the one or more monocytes into one or more IFNα dendritic cells; and loading the one or more IFNα dendritic cells with an antigens presenting composition having one or more cancer cells that are heat shocked and subsequently killed, wherein the loaded one or more IFNα dendritic cells are capable of inducing T cell activation and under conditions that allow the one or more IFNα dendritic cells to present one or more antigens in the composition.

The present invention also includes a cancer specific vaccine capable of inducing T cell activation, includes a pharmaceutically effective amount of one or more dendritic cells activated to present antigen wherein the dendritic cells present one or more antigens that are one or more cancer cells that have been heat shocked and killed after the heat shock. In one embodiment, the cancer cells have been heat shocked or treated and killed (but are not apoptotic) at the time of killing. Another example of the present invention is a melanoma-specific vaccine capable of inducing T cell activation that includes a pharmaceutically effective amount of one or more monocytes exposed to IFNα and that have matured into dendritic cells (IFNα dendritic cells) loaded and presenting an antigen that is heat shocked and subsequently killed melanoma cells, wherein the one or more IFNα dendritic cells are incubated under conditions that promote the presentation of the one or more melanoma to T cells. The vaccine may be made by a method that also includes the step of adding a pulsed preparation for T-cell activation comprising one or more pulsed enucleated antigen presenting cells.

Yet another method of the present invention includes a method of treating a patient suspected of having cancerous cell growth by loading one or more cancer antigens on an activated dendritic cell under conditions that include antigen presentation and that induce T-cell activation, wherein the cancer antigens include cancer cells that have been heat shocked and subsequently killed; and administering to the patient in need thereof the one or more dendritic cells that present one or more cancer antigens to activate T cells.

Yet another method of the present invention includes a method of treated a patient suspected of having cancer by isolating and maturing one or more monocytes with IFNα and GM-CSF into dendritic cells matured with these cytokines, loading the one or more dendritic cells capable of inducing T cell activation with a composition with one or more heat treated cancer cells that are heat shocked and subsequently killed to form one or more antigen presenting dendritic cells; and administering the one or more antigen presenting dendritic cells to the patient in need thereof.

### BRIEF DESCRIPTION OF THE DRAWINGS

For a more complete understanding of the features and advantages of the present invention, reference is now made to the detailed description of the invention along with the accompanying figures and in which:
Figure 1 shows that IFN-DCs efficiently activate cytolytic CD8+T cells. Naive CD8 T cells were cultured for 5 days with IFN-DCs (top) or TNF-DCs (bottom). T cells were stained with anti-CD8 (ordinate) and either anti-Granzyme A (left) or anti-Granzyme B (right). Percentages of double positive CD8⁺T cells.
Figure 2 shows that IFN-DCs respond differentially to maturation stimuli in vitro. Normalized expression of cytokine secretion into supernatants of IFN-DCs exposed for 6 hours to indicated stimuli. Scale 0 (blue) -20 (red). Multiplex cytokine beads analysis of indicated cytokines. Ctrl= no activator was added.
Figure 3 shows that IFN-DCs uniquely secrete IL-7. Details as in Figure 1. IL-7 secretion (ordinate, pg/ml) at 6 hours. Red bars (IFN-DCs); green bars (IL-4 DCs).
Figure 4: IFN-DCs efficiently cross-prime tumor-specific CTLs. Naive CD8⁺T cells were exposed to IL4-DCs and IFN-DCs that were loaded with killed tumor cells. CTL function (ordinate) was measured after two stimulation cycles.
Figure 5 shows that IFN-DCs efficiently cross-prime CTLs against melanoma. HLA-A*0201⁺ naïve CD8⁺T cells were cultured with IFN-DCs loaded with killed Me290 melanoma cells.
Cultures are boosted once at day 7 and 5 days later harvested T cells are assed for their capacity to trigger chromium release from Me290 cells and from control K562 cells (ordinate, specific lysis) at indicated E;T ratios (abscissa).
Figure 6 shows that IFN-DCs efficiently cross-prime melanoma-specific CTLs. Frequency of tetramer specific CD8⁺T cells in cultures with IFN-DCs loaded with either killed melanoma cells (Me290) or melanocytes. HIV tetramer is used as negative control. Values in bold indicate the percentage of CD8⁺T cells binding tetramer.
Figure 7 shows that heat treatment of melanoma cells increases HSP70. SK Mel28 melanoma cells were heated and killed with BA. Cells were mounted onto poly-lysine pretreated slides, fixed and permeabilized. Primary anti-HSP70 Ab was followed by Texas-Red conjugated goat anti-mouse IgG. Leica TCS-NT SP confocal microscopy (x40).
Figure 8 shows that heat-treated melanoma cells display increased immunogenicity: killing of Me275 cells and control K562. Unloaded DCs (CTL 1), DCs loaded with control melanoma bodies (CTL 2) and DCs loaded with heat-shocked melanoma bodies (CTL 3). 14 days of priming. ⁵¹Cr release (ordinate).
Figure 9 shows that heat treated melanoma cells display increased immunogenicity: killing of T2 cells pulsed with a mix of four melanoma peptides (4P; MART-1, gp100, tyrosinase and MAGE-3) or with control PSA peptide. DCs loaded with control melanoma bodies (CTL 1) and DCs loaded with heat-shocked melanoma bodies (CTL 2). ⁵¹Cr release (ordinate).
Figure 10 shows the priming of melanoma-specific CD8⁺T cells by DC loaded with heat-treated killed melanoma cells. a) Tetramer staining after 2 wks culture, and b) after single boost with peptide pulsed DCs. See Figure 7 and text.
Figure 11 shows the heat treatment of melanoma cells enhances transcription of tumor antigens. Real-time PCR: cold (non), heated and heated + Actinomycin D treated Me290 melanoma cells. Normalized fold expression (ordinate).
Figure 12 shows the cross-priming against MAGE A10 up-regulated by heat in killed tumor cells. DCs are loaded with unheated (cold) or heated (hot) killed melanoma cells, either HLA-A*0201⁺ Me290 or HLA-A*0201 negative Skmel 28. After 2 stimulations, HLA-A*0201⁺ CD8⁺T cells are boosted with peptide pulsed DCs. Flow cytometry staining with MAGE 10 tetramer. % of tetramer binding CD3⁺T cells. Two studies.
Figure 13 shows the loading DCs with heat-treated melanoma bodies results in decreased IL-10 secretion and increased IFN gamma secretion by both CD8⁺ (top) and CD4⁺T cells (bottom). Luminex analysis of supernatants at indicated time after the 1^{st} or the 2^{nd} round of stimulation with DCs. Ctrl are unloaded DCs.
Figure 14 is a flow chart that outlines the overall vaccine manufacture process of the present invention;
Figure 15 shows detailed steps and a timeline for the manufacture of the vaccine of the present invention;
Figure 16 shows the morphology of frozen vaccine after thawing;
Figure 17 shows the phenotype of frozen IFN-DC vaccine after thawing;
Figure 18 is a graph that shows the cytokine secretion profile by frozen vaccine after thawing; and
Figure 19 shows the autologous CD8⁺T cell priming by frozen vaccine, Percentage of CD8⁺T cells binding control and MART-1 specific tetramer after two stimulations with MART-1 peptide pulsed frozen/thawed IFN-DCs.

### DETAILED DESCRIPTION OF THE INVENTION

While the making and using of various embodiments of the present invention are discussed in detail below, it should be appreciated that the present invention provides many applicable inventive concepts that can be embodied in a wide variety of specific contexts. The specific embodiments discussed herein are merely illustrative of specific ways to make and use the invention and do not delimit the scope of the invention.

To facilitate the understanding of this invention, a number of terms are defined below. Terms defined herein have meanings as commonly understood by a person of ordinary skill in the areas relevant to the present invention. Terms such as "a", "an" and "the" are not intended to refer to only a singular entity, but include the general class of which a specific example may be used for illustration. The terminology herein is used to describe specific embodiments of the invention, but their usage does not delimit the invention, except as outlined in the claims.

Abbreviations. GM-CSF: Granulocyte macrophage-colony stimulating factor; IFN alpha: Interferon alpha; LPS: lipopolysaccharide; TNF: Tumor necrosis factor; CR: Complete response; PR: Partial response; SD: Stable disease; PD: Progressive disease; NPD: Non-progressive disease; NED: No evidence of disease; MDCs: Monocyte derived dendritic cells; CTLs: Cytotoxic T lymphocytes; NK cells: Natural Killer Cells; NKT cells: Natural Killer T cells; ND: Not done; NT: Not tested; TBD: To be determined.

Cancer immunotherapy. There are numerous strategies for improving a patient's resistance to cancer. Among these strategies are 1) non-specific activation of the immune system with microbial components or cytokines; 2) antigen-specific adoptive immunotherapy with antibodies and/or T cells; and 3) antigen-specific active immunotherapy (vaccination). The major limitation of antibodies is that target proteins must be expressed on the cell surface as opposed to targets for T cells that can be intracellular proteins whose peptides are presented on the cell surface in complexes with MHC molecules¹¹. The identification of defined tumor antigens in humans¹², ¹³ prompted the development of adoptive T-cell therapy. Yet, vaccination remains the most attractive strategy because of its expected inducement of both therapeutic T-cell immunity (tumor-specific effector T cells) and protective T cell immunity (tumor-specific memory T cells that can control tumor relapse)¹⁴⁻¹⁶.

Numerous approaches for the therapeutic vaccination of humans with cancer have been developed, including: autologous and allogeneic tumor cells (which are often modified to express various cytokines), peptides, proteins and DNA vaccines ^{8, 16-19}. All of these approaches rely on a random encounter of the vaccine with host DCs. If the vaccine antigen does not result in an encounter with DCs an immune response cannot occur. In addition, an inappropriate encounter with either non-activated DCs or the "wrong" subset of DCs can lead to silencing of the immune response²⁰. Both of these scenarios may explain some of the shortcomings in current cancer vaccines. Furthermore, it is not known how tumor antigens need to be delivered to DCs *in vivo* in order to elicit an appropriate immune response. Hence, there is a need for studies based on *ex vivo*-generated autologous DCs that are loaded with tumor antigen under controlled conditions which might allow the establishment of parameters for optimal vaccination against cancer. Clinical studies using DCs as vaccines were facilitated by the discovery of methods to generate large numbers of autologous DCs *ex vivo* ²¹⁻²³. Parameters that should be considered to improve the efficacy of DC vaccinations in cancer include: DC related factors; host-related factors; and a combination of DC vaccines with other therapies. Two DC related parameters, i.e., a novel DC subset and a novel strategy to load tumor antigens are briefly discussed below.

Parameters of DC vaccines. DC subsets: The discovery that GM-CSF and IL-4 can differentiate monocytes into immature DCs²²⁻²⁴ has allowed major progress in our understanding of DC biology and function. Several institutions have used IL4-DCs as vaccines⁸ following pioneering clinical studies in patients with metastatic melanoma by Nestle, et al.²⁵ (using tumor-lysate-loaded DCs) and by Schuler and colleagues²⁶ (using melanoma-peptide-loaded DCs). However, recent discoveries point to new alternatives to the classical way of generating DCs. For example, melanoma-peptide-pulsed IL15-DCs are more efficient than IL4-DCs for the induction of antigen-specific CTL differentiation *in vitro,* whereas their ability to stimulate CD4⁺ T cells is comparable²⁷. Also, IFN-alpha-DCs generated in three-day cultures have been found to be efficient for the induction of specific immunity²⁸. Thus, the immunogenicity of these distinct DC vaccines warrants testing *in vivo* in clinical studies.

Antigen loading. Loading MHC class I and MHC class II molecules on DCs with peptides derived from defined antigens is the most commonly used strategy for DC vaccination^{15,29}. Although this technique is important for "proof of concept" studies, the use of peptides has limitations for further vaccine development: restriction to a given HLA type; the limited number of well characterized tumor-associated antigens (TAA); the relatively rapid turnover of exogenous peptide-MHC complexes resulting in comparatively low antigen presentation at the time the DC arrive into draining lymph node after injection; and, the induction of a restricted repertoire of T-cell clones, thus limiting the ability of the immune system to control tumor antigen variation. Many of these peptides may differ from naturally processed epitopes. Thus, loading DCs with total antigen preparations and allowing "natural" processing and epitope selection is expected to improve efficacy and allow the generation of a diverse immune response involving many clones of CD4⁺ T cells and CTLs. All of these strategies load DCs with recombinant proteins, exosomes³⁰, viral vectors³¹, plasmid DNA, RNA transfection³², immune complexes³³ and antibodies specific for DC surface molecules^{15,34}.

Another technique involves exploiting the capacity of DCs to present peptides from phagocytosed dead tumor cells on MHC class I molecules (as well as class II molecules). This is known as cross-priming^{35, 36}. Indeed, DCs cultured with killed allogeneic melanoma, prostate or breast cancer cell lines prime naïve CD8⁺ T cells against tumor antigens *in vitro* ^{36,37}. 20 patients with metastatic melanoma have been vaccinated at BIIR to date with autologous monocyte-derived DCs previously exposed to a killed allogeneic melanoma cell line (8 vaccines on a monthly basis). Vaccination has proved to be safe (no autoimmunity or other adverse events) and has led to the induction of melanoma-specific T cell immunity. In two patients, a long-lasting tumor regression has been observed. These results warrant larger clinical studies to prove the efficacy of the vaccine preparation methodology.

GM-CSF and IFN alpha induced dendritic cell vaccine. Several recent findings in IFN biology reactivated immunologists' interest in this family of molecules. These findings include 1) the demonstration that plasmacytoid dendritic cells (pDCs, a subset of human and murine DCs) promptly secrete large amounts of type I IFNs in response to viral signals⁷⁴; 2) the abilities of IFN alpha/beta to activate immature myeloid DCs⁷⁵ and to induce the differentiation of monocytes into DCs^{28,76}; 3) the activity of IFN alpha/beta in the generation of memory CD8+T cells^{77,78} and the stimulation of antibody responses^{5, 79, 80}; 4) the central role of IFN alpha/beta in the pathogenesis of Systemic Lupus Erythematosus^{81, 82}; 5) the secretion of low levels of IFN alpha/beta to rev up the immune system, as illustrated by the essential role of IFN alpha/beta in the LPS signaling and the development of septic shock.⁸³

It was recently recognized by the present inventors that IFN alpha is essential in launching human humoral responses specific to Influenza virus.⁵ This raised a potential that IFN alpha could possibly enhance the generation of CTLs, most particularly those directed against tumor antigens.

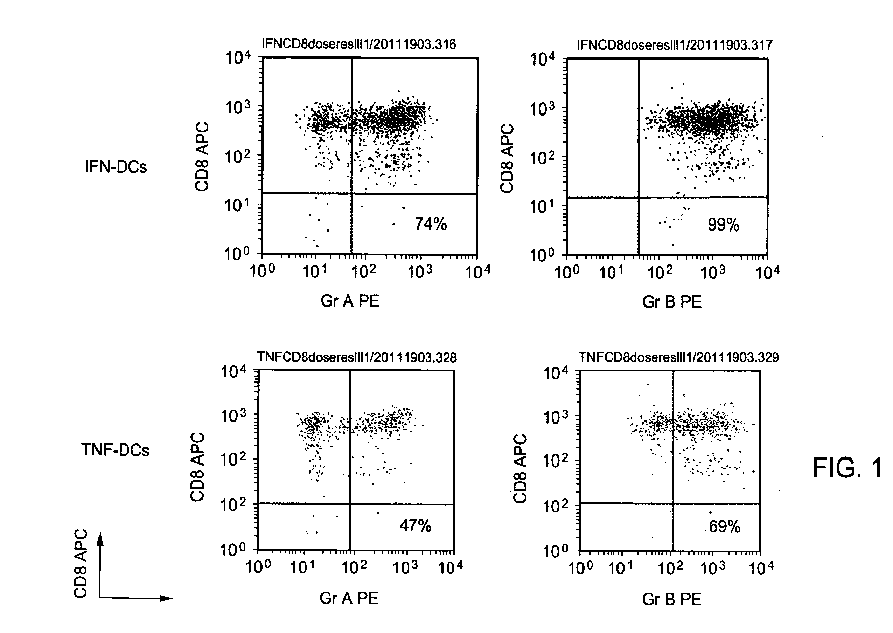

IFN-DCs efficiently activate cytolytic CD8⁺T cells: The biological properties of DC vaccine generated from monocytes by culturing them with GM-CSF and IFN alpha (IFN-DC) were compared with two previous products manufactured, viz., DC vaccines made by culturing monocytes with GM-CSF and either TNF (TNF-DCs) or IL-4 (IL4-DCs). Allogeneic CD8⁺T cells were cultured for 5 days with each DC subset, then re-purified by cell sorting and analyzed by flow cytometry. Strikingly, results showed that IFN-DCs can induce CD8⁺ T cells with considerably higher expression of cytolytic T cell molecules such as Granzymes A and B (Figure 1) and perforin (not shown).

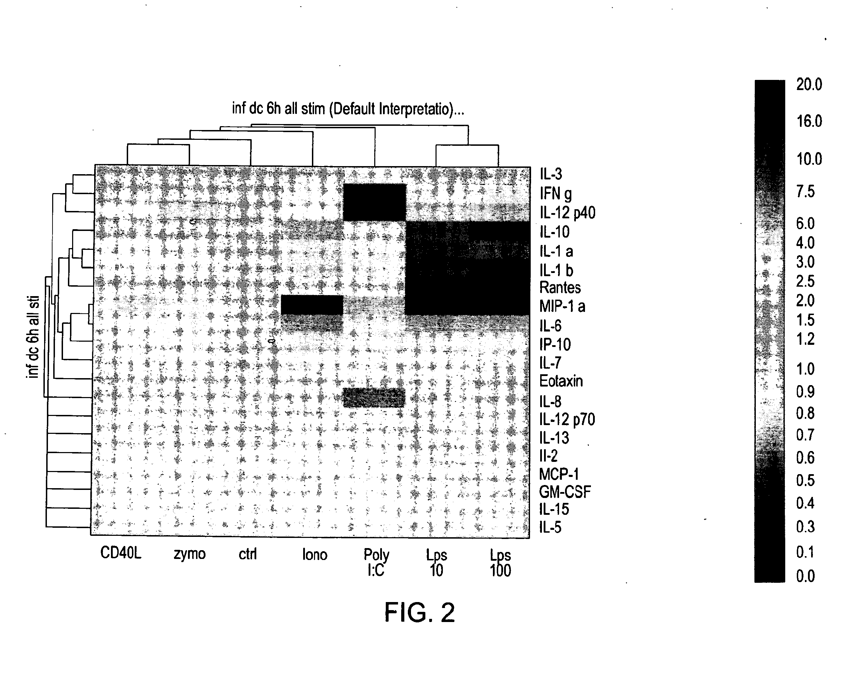

IFN-DCs respond differentially to maturation stimuli: To further characterize the role of IFN-DCs in cancer vaccines, cytokine biosignatures were analyzed in IFN-DCs exposed *in vitro* to various activation signals including CD40 ligand, Ionomycin, and TLR ligands such as LPS (TLR4), poly I:C (TLR3) and zymosan (TLR2). IFN-DCs, generated by culturing purified monocytes with GM-CSF and recombinant human IFN alpha 2a, were exposed to various stimuli and cytokine/chemokine secretion into culture supernatants and measured at different time points using Multiplex cytokine beads (Luminex). Preliminary analysis demonstrated that the profile of secreted cytokines does not change significantly over 48hrs of exposure (not shown). As shown in Figure 2, the predominant cytokine response was triggered by LPS, followed by poly I:C and Ionomycin. Only minor cytokine secretion was induced upon exposure to CD40 ligand or Zymosan. These preliminary results demonstrate different activation signals trigger different cytokine signatures in IFN-DCs.

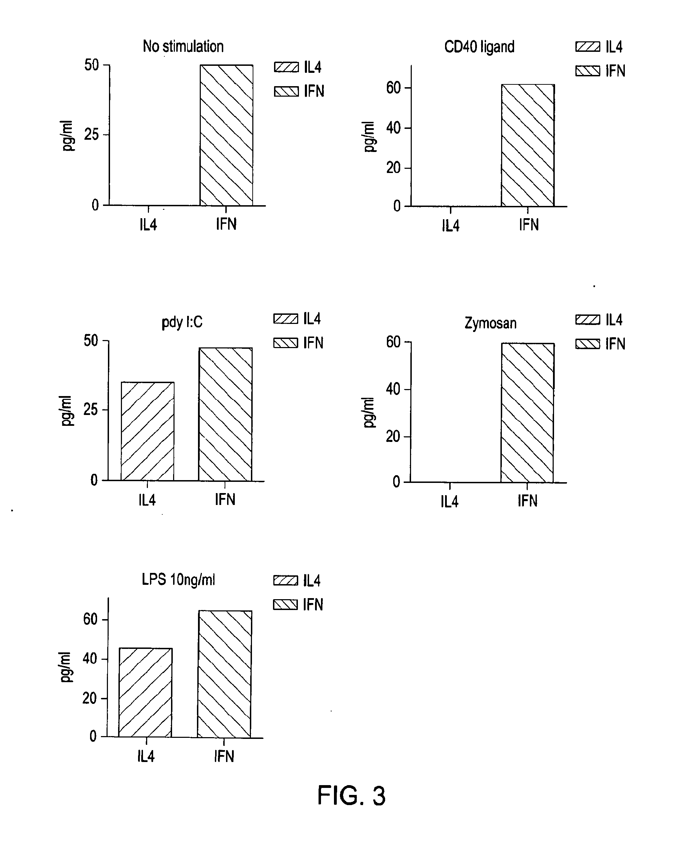

IFN-DCs uniquely secrete IL-7, a T cell growth factor: Upon single cytokine analysis, it was observed that IFN-DCs spontaneously secrete detectable levels of IL-7 (Figure 3) while non-activated IL4-DCs were unable to do so. However, activation of IL-4DCs with signals known to activate Type I interferon pathway (lipopolysaccharide (LPS) and poly I:C) but not with CD40 ligand or Zymosan, led to induction of IL-7 secretion (Figure 3). These results yield two important conclusions for T cell activation: 1) IL-7 secretion by IFN-DCs could have a role in their superior capacity to prime naïve CD8⁺T cells, and 2) Type I interferon appears to regulate IL-7 secretion by myeloid DCs.

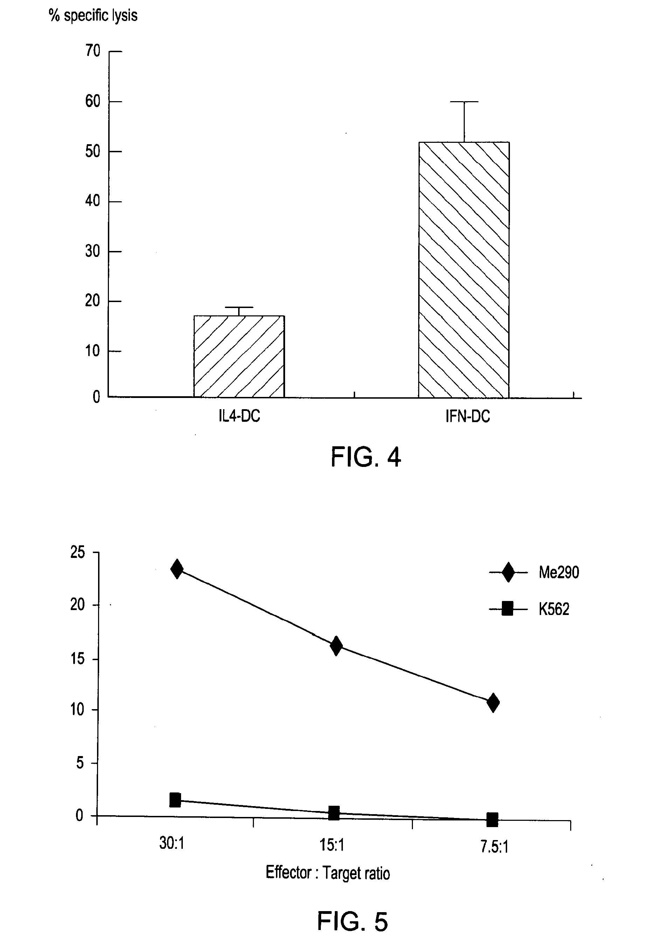

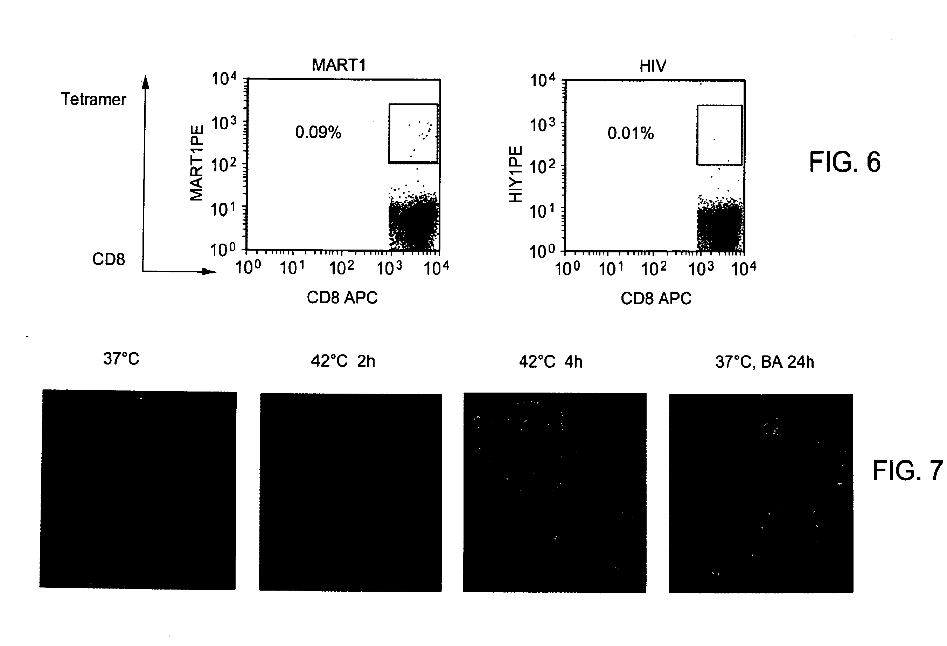

IFN-DCs efficiently cross-prime tumor-specific CTLs: To determine whether IFN-DCs were indeed more powerful in stimulating CTLs, DCs were loaded with killed melanoma or breast cancer cells and then used to prime purified CD8⁺T cells in 2 culture cycles. IFN-DCs were observed to be more efficient than IL4-DCs in priming CTLs that have the capacity to kill cancer cells (Figure 4). To assess the capacity of IFN-DCs to prime naive CD8⁺T cells, we used an *in vitro* cross-priming system against tumor antigens that we had established earlier. In this situation, DCs are loaded with killed allogeneic tumor cells and used to prime autologous naive CD8⁺T cells over two-week cultures. As shown in Figure 5, IFN-DCs loaded with killed allogeneic HLA-A*0201+ Me290 melanoma cells are remarkably efficient in priming CTLs with the ability to kill Me290 cells used for immunization. The presence of melanoma-specific CD8⁺T cells was further confirmed by the analysis of tetramer specific T cells. As shown in Figure 6, the elicited CD8⁺T cells contain a subpopulation of MART-1 specific T cells.

Thus, IFN-DCs have been shown to be highly efficient at cross-priming naive CD8+T cells to differentiate into CTLs specific for tumor antigens. This finding has potentially high therapeutic implications for cancer vaccines. Thus, IFN-DCs are more efficient in the induction of tumor specific immunity which warrants further testing of their in vivo activity in patients in the clinical setting.

Generation of highly immunogenic killed tumor cells for loading DC vaccines. The use of hyperthermia in cancer therapy, either alone or as an adjuvant for radiotherapy, has been an object of clinical interest for many years ⁸⁴. Hyperthermia seems to be particularly effective in combination with radiotherapy and/or radio-immunotherapy (reviewed in ^{85,86}). The molecular mechanism by which hyperthermia leads to radiosensitization is not clear, however activation of early response genes and heat shock factors (HSFs) and subsequently heat shock proteins (HSPs) are likely to play a role in this occurrence⁸⁷. HSPs constitute a superfamily of distinct proteins, which are operationally named according to their molecular weight, e.g. hsp70. Most HSPs are expressed constitutively and are further induced under stress conditions, including temperature increase. HSPs are considered as molecular relay line that chaperones the peptides from their generation in the cytosol to their binding to MHC class I in the ER ^{88,89}. HSP70, HSP60 and GP96 have been recently established as immune adjuvants for cross-priming with antigenic proteins or peptides ^{90,91}. In this process, reconstituted hsp70 or gp96-peptide complex are internalized by antigen presenting cells (APCs) including DCs, through receptor-mediated endocytosis via CD91⁹², CD40⁹³, LOX-1⁹⁴ or TLR2/4⁹⁵. Thus, the present inventors recognized that hyperthermia could enhance cross-priming and thereby contribute to the manufacture of a vaccine which would allow enhanced tumor regression.

Heated Melanoma Cells. The goal of current research has been to create altered tumor cell bodies that are highly immunogenic and could be used to load DCs for vaccination purposes.

Therefore, having established the premise that melanoma cell lines overexpressing HSP70 are indeed more immunogenic (not shown), the focus of this investigation now shifted to the development of a means to increase HSP expression in clinical grade conditions. Thus, whether heat-treatment of melanoma cells could increase immunogenicity of loaded DCs was determined.

Heat treatment of melanoma cells increases HSP70: Melanoma cell lines were incubated in the for 4 hrs at 42°C (heat shock). The analysis of HSPs expression by ELISA (not shown) or by intracellular staining (Figure 7) revealed, as expected, significant upregulation of HSP70⁸⁵. Killed melanoma cells (melanoma bodies) were generated from either untreated or heat-shock treated cells by introduction of betulinic acid (BA).

DCs loaded with heat-treated melanoma bodies rapidly yield CTLs able to kill melanoma cells: Unloaded DCs (CTL 1), DCs loaded with control melanoma bodies (CTL 2) and DCs loaded with heat-shocked melanoma bodies (CTL 3) were cultured for 2 weeks with purified naïve CD8+ T cells. T cell cultures were restimulated once (a total of two stimulations) and were supplemented with soluble CD40 ligand and low dose IL-7 (10 U/ml, all culture) and IL-2 in the second week (10 U/ml). T cells were analyzed 7 days after restimulation (total 14 days of culture).

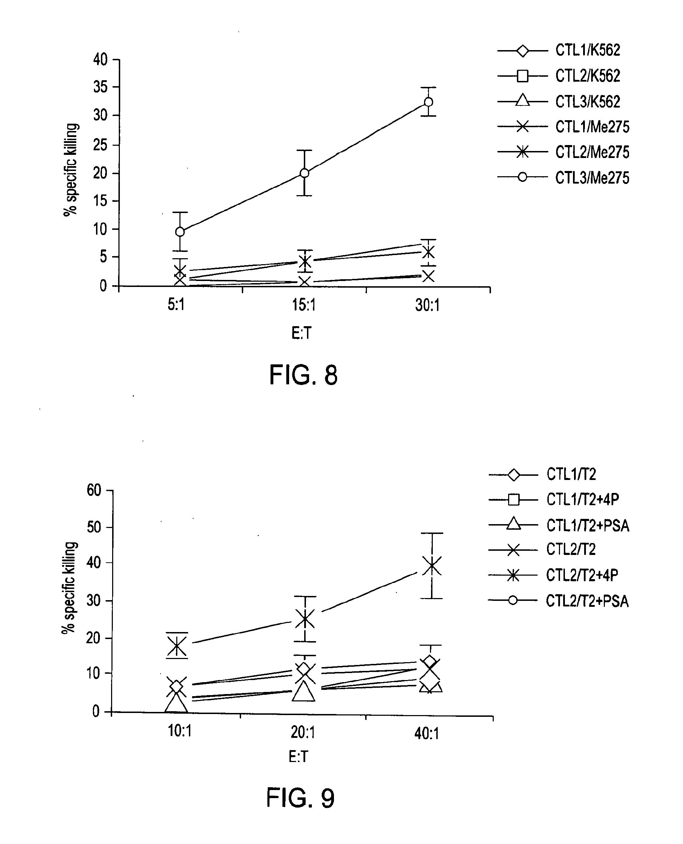

It was noted that T cells cultured with DCs loaded with heat- treated Me275 melanoma bodies but not control bodies, were able to kill Me275 cells (Figure 8). The killing was specific to T cells as no lysis of K562 was found. Furthermore, after two stimulations, the T cells were able to kill another HLA-A*0201 melanoma cell line (Me290) (not shown), suggesting cross-priming against shared antigens can occur. The killing was specific to melanoma cells as HLA-A*0201 MCF7 breast cancer cells were not lysed (not shown). Finally, killing of melanoma cells was at least partially restricted by the expression of MHC class I, as the pretreatment of target cells with MHC class I blocking mAb W6/32 resulted in >60% inhibition of Me275 killing at different E:T ratios (not shown).

DCs loaded with heat shocked melanoma bodies rapidly yield melanoma-specific CTLs: Naïve CD8⁺T cells primed with heat-treated melanoma bodies can specifically and efficiently kill T2 cells loaded with four melanoma peptides but not PSA peptide. Melanoma cell lines were also destroyed, but the breast cell line MCF7 and K562 cells were unaffected (Figure 9). DCs loaded heat-treated killed melanoma cells efficiently educated naive CD8+T cells to become melanoma-specific cytotoxic T cells.

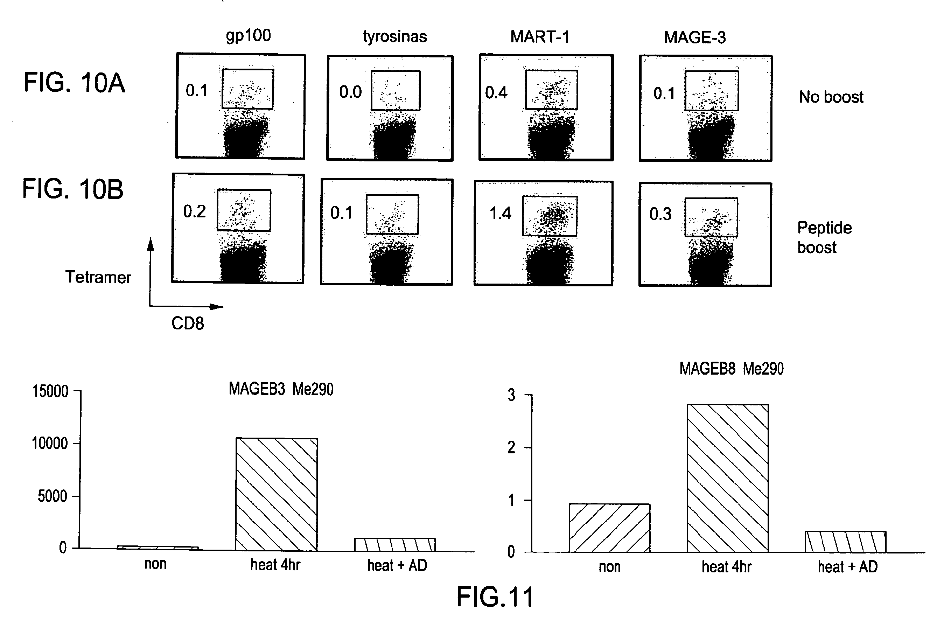

Tetramer binding analysis confirmed this finding and showed that up to 0.4% of CD8⁺T cells were specific for MART-1 (Figure 10). However, other specificities were barely detected. Thus, 7 days after the 2^{nd} stimulation, the T cells were restimulated with autologous DCs pulsed either with each of the four melanoma peptides, or with a control PSA peptide. These cultures were analyzed after 7 days of culture. Results showed that boosting with melanoma peptide-pulsed DCs expanded melanoma-specific CD8⁺ T cells (Figure 10) while no increased expansion was observed in the boost with control peptide (not shown). Thus, heat treatment of melanoma cells results in enhancement of melanoma-specific responses.

Heat treatment of melanoma cells enhances transcription of tumor antigens. The original hypothesis that the enhanced cross-priming would be due to enhanced expression of Heat Shock Proteins was found to be consistent with studies that demonstrated purified HSP70, HSP60 and GP96 act immune adjuvants for cross-priming with antigenic proteins or peptides ^{90,91}. After Microarray analysis of control and HSP70 transduced Sk-Me128 cells, however, an increased transcription of several tumor antigens including MAGE-A10 (not shown) was observed. Therefore, 12 genes encoding different members of MAGE tumor antigen family were measured by real-time PCR expressions. These antigens included MAGE-B3, MAGE-A8 (Figure 11), MAGE-B4, and MAGE-A10 (not shown). Expression of MAGE-B3 increased up to 10000 fold in addition to sensitivity to Actinomycin D thus confirming active transcription (Figure 11).

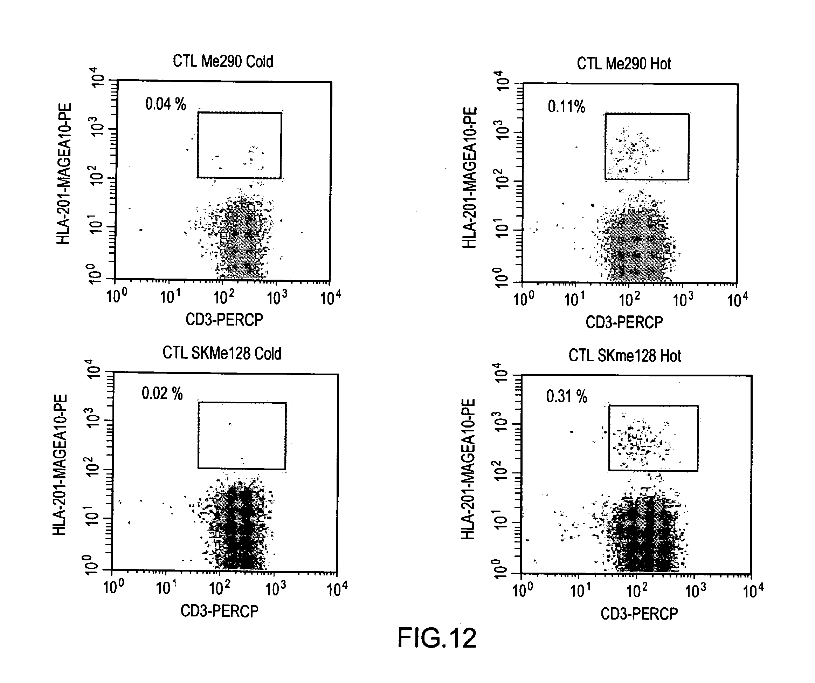

Therefore, HLA-A*0201 restricted peptides derived from MAGE-A8 and MAGE-A10 were identified and analyzed to determine whether DCs loaded with heat-treated bodies could prime CTLs specific for these two epitopes. As shown in Figure 12, HLA-A*0201⁺ CTLs primed against hot HLA-A*0201⁺ Me290 or HLA-A*0201^{neg} Sk-Me128 cells displayed a higher frequency of MAGE-A10 tetramer binding CD8⁺T cells than CTLs primed against unheated melanoma cells.

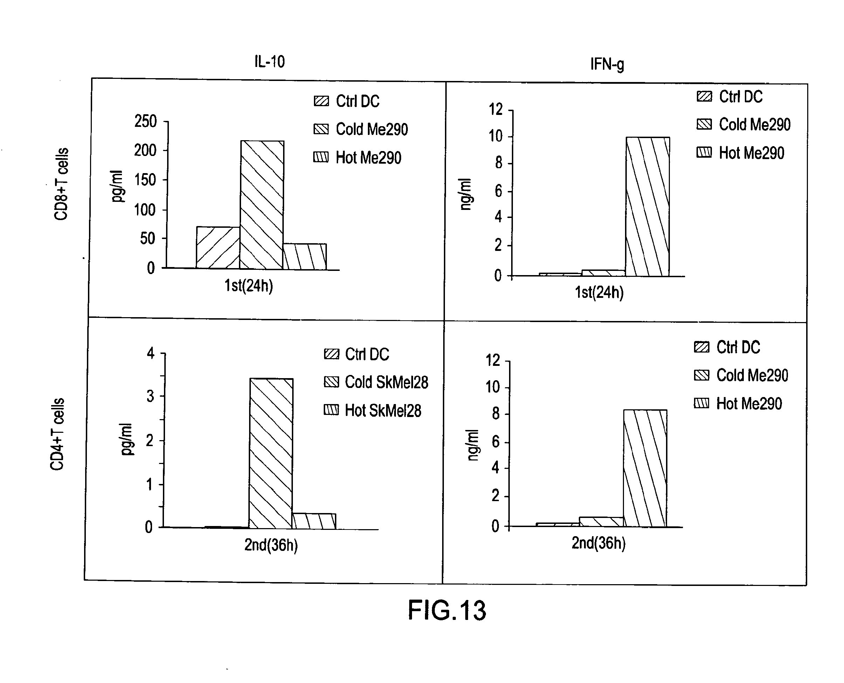

Thus, hyperthermia-increased transcription of tumor antigens could contribute to enhanced cross-priming. DCs loaded with heat-treated killed melanoma bodies induce autologous naïve CD8+ or CD4+T cells to produce increased levels of IFN gamma and decreased levels of IL-10 Regulatory/suppressor T cells are considered to be one of the major obstacles in successful vaccination of patients against their cancer. As shown in Figure 13, loading DC vaccines with heat-treated killed melanoma cells resulted in decreased secretion of IL-10 and increased production of IFN gamma when compared to cultures made with DC loaded with unheated tumor. These results support the unique strategy of DC loading *in vivo* in a clinical trial. Therefore, Loading DC vaccines with heat-treated and killed melanoma cells leads to induction of broad melanoma-specific Type 1 CD8+T cell immunity.

The present invention includes autologous dendritic cells derived from monocytes with GM-CSF and IFN alpha and loaded with killed allogeneic Colo829 melanoma cells. Autologous dendritic cells are manufactured from monocytes separated by elutriation from peripheral blood mononuclear cells obtained by apheresis. Monocytes are cultured in a closed system in the presence of granulocyte-macrophage colony stimulating factor (GM-CSF) and Interferon alpha (IFN alpha, loaded with heated and killed allogeneic COLO 829 tumor cells and cryopreserved. Vaccine is stored in liquid nitrogen (vapor phase).

Dendritic cells are manufactured from the apheresis product which is processed to isolate monocytes using the Elutra system (Gambro BCT). Monocytes are transferred from elutriation bag into cultures bags (100 ml volume each) and cultured at 1 x10⁶/ 1 ml volume for 72 hours. In this example, the culture media included serum-free media supplemented with recombinant human GM-CSF (100ng/ml; Berlex) and Interferon alpha 2b (500 IU/ml; Schering Plough). The skilled artisan, however, would know to titrate the amounts, timing and length of exposure of the cells to the GM-CSF and/or the Interferon alpha to maximize the activation. After initial 24 hours of culture, the vaccine is loaded with killed Colo829 cells. After total of 72 hours culture, dendritic cells are harvested, medium and cytokines are washed out with normal saline. The cells are re-suspended in autologous serum containing 10% DMSO and 10% Plasmalyte, and distributed into cryo-vials at 30 x 10⁶ cells/vial. The cryovials are frozen using an automated rate controlled freezer and stored in liquid nitrogen (vapor phase).

**Table 1: Manufacturing Stages**

| Stage | Description |
|---|---|
| I | ISOLATION-Autologous peripheral blood mononuclear cells are obtained by apheresis using a COBE SPECTRA™. Monocytes are then isolated from the peripheral blood mononuclear cells on a GAMBRO BCT ELUTRA™. |
| II | CULTURE / LOADING-Dendritic cells are manufactured by culturing monocytes with GM-CSF and IFN alpha for 3 days. Twenty-four hours after culture initiation, killed allogeneic tumor cells (COLO 829) are added to the culture. |
| III | HARVESTING/CRYOPRESERVATION-Dendritic cell vaccine will be cryopreserved in aliquots for multiple doses. The vials will then be frozen using a rate controlled freezer. |

Isolation. Apheresis - Collect PBMC. Autologous peripheral blood mononuclear cells are obtained by apheresis using a COBE SPECTRA™.

Elutriation - Isolate monocytes. Monocytes are then isolated from the peripheral blood mononuclear cells on a GAMBRO BCT ELUTRA™. The Elutra system is a semi-automatic, closed system centrifuge that uses continuous counter-flow elutriation technology to separate cells into multiple fractions based on size and density. The Elutra system's intended application is monocyte enrichment. The system automatically collects 5 fractions of cells based on size and density. Fraction 5 contains an enriched monocyte population (∼90% purity, n=7 healthy volunteers apheresis) which is used for dendritic cell vaccine manufacture.

Culture -The bag containing Fraction 5 is centrifuged and the buffered saline is expressed off and discarded. The cells are resuspended in CellGenix DC culture medium and a sample is removed for cell count, viability and phenotype. Once the number of monocytes is determined, the cells are diluted to a concentration of 1 x 10 monocytes / mL and sterile connected to AFC VueLife culture bags. Cytokines GM-CSF (Leukine®) (Berlex Inc.) at a concentration of 100 ng/mL and IFN alfa-2b (INTRON A) (Schering-Plough Corp.) at a concentration of 500 IU/mL are added and the cells are placed in a 37°C 5% CO₂ incubator for culture.

Loading -24 hours after culture initiation, killed tumor cells (COLO829) are added as a source of antigen as well as second dose of GM-CSF and IFN alfa-2b. The killed COLO829 is prepared in batches using gamma irradiation, tested for sterility and inability to proliferate as measured by tritiated thymidine incorporation, frozen at a concentration of 50 x 10⁶ / mL with 10% DMSO in cryovials, and stored in vapor phase nitrogen. An appropriate number of cryovials are thawed so that the DC are loaded 1 killed tumor cell per 2 dendritic cells. The killed COLO829 are washed with culture medium 3 times and added to the culture bags in a small volume.

Dendritic Cell Harvest. 72 hours after culture initiation, the cells are harvested and the vaccine is cryopreserved. The culture bags are centrifuged, the supernatant is expressed and the cells are washed 3 times with normal saline. Washing consists of connecting a bag of normal saline to the culture bag, resuspending the cells in the normal saline, centrifuging the culture bag, and expressing the normal saline into a waste collection bag. After the third wash, the cells are resuspended in Plasmalyte which is the freezing solution diluent. A sample is removed for cell count and viability.

Rate controlled freezing - The vials are frozen using a rate controlled freezer. The vials are placed in a freezing chamber and liquid nitrogen enters the chamber through an electronic solenoid valve. Since vaporization is almost instantaneous, controlling the rate at which liquid nitrogen enters the chamber directly controls the rate at which heat is absorbed and removed from the freezing chamber and its contents.

Cryopreservation. The cells are cryopreserved at a concentration of 30 x 10⁶ cells / mL. Once the cell number is determined the cells are diluted to 2 x the final concentration with autologous serum. A bag of freezing solution containing a volume equal to the cell volume is prepared. The freezing solution is autologous serum with 20% DMSO and 20% plasmalyte. Working rapidly, the freezing solution is added to the cell bag and the cells are transferred to labeled cryovials. The final concentration of DMSO is 10%. The cryovials are frozen using an automated rate controlled freezer at 1°C / min and stored in vapor phase nitrogen.

Storage - The cryovials are transferred from the rate controlled freezer to a liquid nitrogen tank for long term storage. Inventory control is maintained in a database by GMP trained personnel.

**Table 2: Validation Lots Manufactured**

| Lot No. | Location of Manufacture | Date of Manufacture | Manufacturing Scale | Overall Yield of Cells | Use of Lot |
|---|---|---|---|---|---|
| ND66 | BIIR | 05/25/04 | 1/3 of Fraction 5 fresh 2 x 10⁸ | 1.60 x 10⁸ | Process Validation |
| ND68 | BIIR | 05/27/04 | 1/6 of Fraction 5 fresh 3 x 10⁸ | 1.31 x 10⁸ | Process Validation |
| ND70 | BIIR | 06/01/04 | 1/3 of Fraction 5 fresh 4 x 10⁸ | 2.14 x 10⁸ | Process Validation |
| ND71 | BIIR | 06/02/04 | 1/9 of Fraction 5 fresh 4 x 10⁸ | 3.39 x 10⁸ | Process Validation |
| ND72 | BIIR | 06/03/04 | 1/4 of Fraction 5 fresh 4 x 10⁸ | 1.43 x 10⁸ | Process Validation |
| ND73 | BIIR | 06/08/04 | 1/6 of Fraction 5 fresh 4 x 10⁸ | 1.95 x 10⁸ | Process Validation |
| ND80 | BIIR | 10/21/04 | Fraction 5 thawed 51 x 10⁶ | 23 x 10⁶ | Process Validation |
| ND80 | BIIR | 10/28/04 | Fraction 5 thawed 46 x 10⁶ | 34 x 10⁶ | Process Validation |
| ND84 | BIIR | 11/22/04 | 10% of Fraction 5 fresh 2.5 x 10⁸ | 153 x 10⁶ | Process Validation |
| ND84 | BIIR | 12/06/04 | Fraction 5 thawed 44 x 10⁶ | 13 x 10⁶ | Process Validation |
| ND85 | BIIR | 12/09/04 | 5% of Fraction 5 fresh 1.0 x 10⁸ | 61 x 10⁶ | Process Validation |
| ND86-1 | BIIR | 12/19/04 | 5% of Fraction 5 fresh 1.0 x 10⁸ | 100 x 10⁶ | Process Validation |
| ND86-2 | BIIR | 12/20/04 | 1/3 of Fraction 5 fresh 0.5 x 10⁸ | 39 x 10⁶ | Process Validation |
| ND86-1 | BIIR | 01/10/05 | Fraction 5 thawed 25 x 10⁶ | 2 x 10⁶ | Process Validation |
| ND89 | BIIR | 01/15/05 | 1/3 of Fraction 5 fresh 7 x 10⁸ | 6.8 x 10⁸ | Process Validation |
| ND90 | BIIR | 02/04/05 | 1/3 of Fraction 5 fresh 3 x 10⁸ | 2.6 x 10⁸ | Process Validation |

Stability of Final Dosage Form of the Active Pharmaceutical Ingredient. Description: Vials containing 1 mL of cryopreserved cells were quickly thawed at 37° and immediately drawn into a syringe containing 9 mL of sterile normal saline (the condition in which cells will be thawed at the clinical site immediately prior to injection). The normal saline was either at room temperature or 4°C (refrigerated). The cells were then evenly distributed among 6 tubes and kept at either room temperature or refrigerated. The diluted cells were then assayed at various time points.

**Table 3: Product viability as a function of time post-thaw**

| | % VIABILITY | | | | | |
|---|---|---|---|---|---|---|
| | 0 min | 15 min | 30 min | 45 min | 60 min | 75 min |
| EXP 1 | 85 | 87 | 85 | 88 | 87 | 78 |
| EXP 2 | 88 | 87 | 78 | 90 | 88 | 86 |
| EXP 3 | 89 | 92 | 88 | 88 | 93 | 80 |
| EXP 4 | 91 | 89 | 89 | 86 | 86 | ND |
| EXP 5 | 87 | 87 | 82 | 86 | 86 | ND |
| EXP 6 | ND | ND | ND | ND | 86 | ND |
| EXP 7 | ND | ND | ND | ND | 86 | ND |
| EXP 8 | ND | ND | ND | ND | 88 | ND |
| EXP 9 | ND | ND | ND | ND | 94 | ND |
| EXP 10 | ND | ND | ND | ND | 82 | ND |
| EXP 11 | ND | ND | ND | ND | 72 | ND |
| MEAN | 88.0 | 88.4 | 84.4 | 87.6 | 86.2 | 81.3 |
| STD DEV | 2.24 | 2.19 | 4.51 | 1.67 | 5.78 | 4.16 |

**Table 4: Product recovery: viable cells (relative to cell number per vial prior to freezing) as a function of time post-thaw**

| | % RECOVERY OF VIABLE CELLS | | | | | |
|---|---|---|---|---|---|---|
| | 0 min | 15 min | 30 min | 45 min | 60 min | 75 min |
| EXP 1 | 100 | 60 | 68.5 | 32 | 36 | 55 |
| EXP 2 | 95 | 55 | 25 | 49.6 | 34 | 35 |
| EXP 3 | 60 | 82 | 86.6 | 38 | 34 | 16.3 |
| EXP 4 | 85 | 62.5 | 59 | 70 | 55 | ND |
| EXP 5 | 55 | 46 | 43 | 55 | 45 | ND |
| EXP 6 | ND | ND | ND | ND | 42 | ND |
| EXP 7 | ND | ND | ND | ND | 36 | ND |
| EXP 8 | ND | ND | ND | ND | 31 | ND |
| EXP 9 | ND | ND | ND | ND | 86 | ND |
| EXP 10 | ND | ND | ND | ND | 89 | ND |
| EXP 11 | ND | ND | ND | ND | 98 | ND |
| MEAN | 79.0 | 61.1 | 56.4 | 48.9 | 53.3 | 35.4 |
| STD DEV | 20.43 | 13.28 | 23.62 | 14.89 | 25.26 | 19.35 |

**Table 5: Product potency in mixed lymphocyte reaction with CD4+T cells**

| | | | |
|---|---|---|---|
| | | MLR (cpm) | |

| | 5000 DC | 2000 DC | 500 DC |
|---|---|---|---|
| EXP 1 | 262,340 | 245,887 | 189,296 |
| EXP 4 | 194,437 | 91,980 | 22,127 |
| EXP 5 | 69,407 | 47,926 | 36,717 |
| MEAN | 175,395 | 128,598 | 82,713 |
| STD DEV | 97,866 | 103,936 | 92,591 |

**Table 6: Product recovery and viability when thawed at room temperature and at 4°C.**

| **A** | % RECOVERY | | | | | |
|---|---|---|---|---|---|---|
| | ROOM TEMP | | | 4°C | | |
| | MEAN | STD DEV | RANGE | MEAN | STD DEV | RANGE |
| 0 min | 79.0 | 20.43 | 55.0 - 100.0 | 49.5 | 14.84 | 35.0 - 65.0 |
| 15 min | 61.1 | 13.28 | 46.0 - 82.0 | 38.0 | 22.03 | 7.3 - 63.5 |
| 30 min | 56.4 | 23.62 | 25.0 - 86.6 | 40.5 | 25.76 | 15.0 - 80.0 |
| 45 min | 48.9 | 14.89 | 32.0 - 70.0 | 52.9 | 39.57 | 9.6 - 115 |
| 60 min | 40.8 | 9.15 | 34.0 - 55.0 | 49.5 | 47.40 | 7.3 - 126 |

| **B** | % VIABILITY | | | | | |
|---|---|---|---|---|---|---|
| | ROOM TEMP | | | 4° C | | |
| | MEAN | STD DEV | RANGE | MEAN | STD DEV | RANGE |
| 0 min | 88.0 | 2.24 | 85 - 91 | 88.6 | 3.36 | 85 - 94 |
| 15 min | 88.4 | 2.19 | 87 - 92 | 81.2 | 14.24 | 56 - 90 |
| 30 min | 84.4 | 4.51 | 78 - 89 | 85.8 | 3.27 | 81 - 90 |
| 45 min | 87.6 | 1.67 | 86 - 90 | 87.8 | 3.83 | 83 - 93 |
| 60 min | 88.0 | 2.92 | 86 - 93 | 85.8 | 4.15 | 80 - 91 |

Acceptable results for lot release are at least 50% recovery of viable cells and at least 50% viability at 15 min after thaw at room temp. Release testing is performed on three vials obtained at the beginning (1^{st}), in the middle (2^{nd}), and in the end (3^{rd}) of the freezing process.

Frozen vaccine characterization. To further characterize frozen vaccine, beyond viability and capacity to stimulate MLR with CD4+T cells as described above, one or more of the following may be analyzed: (1) morphology and phenotype; (2) cytokine secretion; and/or (3) capacity to induce autologous CD8⁺T cell differentiation.

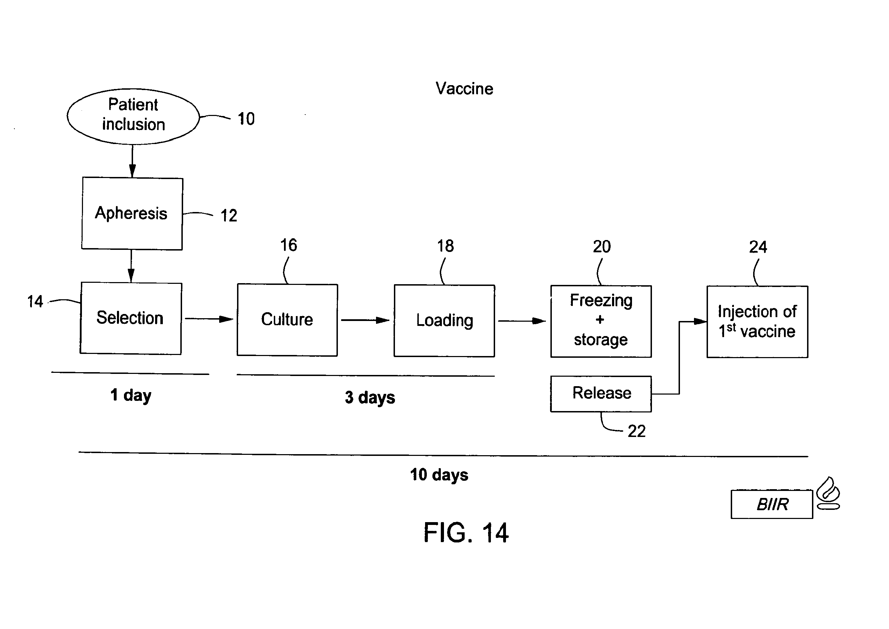

Figure 14 is a flow chart that outlines the overall vaccine manufacture process of the present invention. In step 10, a patient is selected for inclusion in the vaccine production process. In step 12, blood apheresis of the patient is conducted to isolate the cells for loading are obtained and selected in step 14. Steps 10, 12 and 14 may be conducted in Day 1. In the next few days, the cells are cultured (step 16) and loaded (step 18) with the antigen(s) that are presented by the antigen-loaded dendritic cells obtained and cultures in step 16. Next, in step 20 the cells may be frozen and stored for future use and/or eventually thawed and released in step 22. Finally, the cell vaccines may be used for injection in step 24. In one embodiment, the entire process may occur in about 10 days.

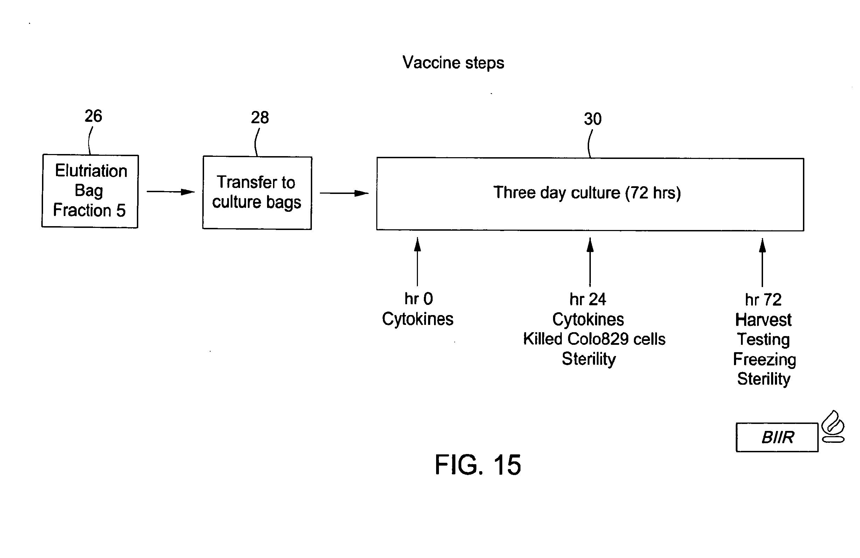

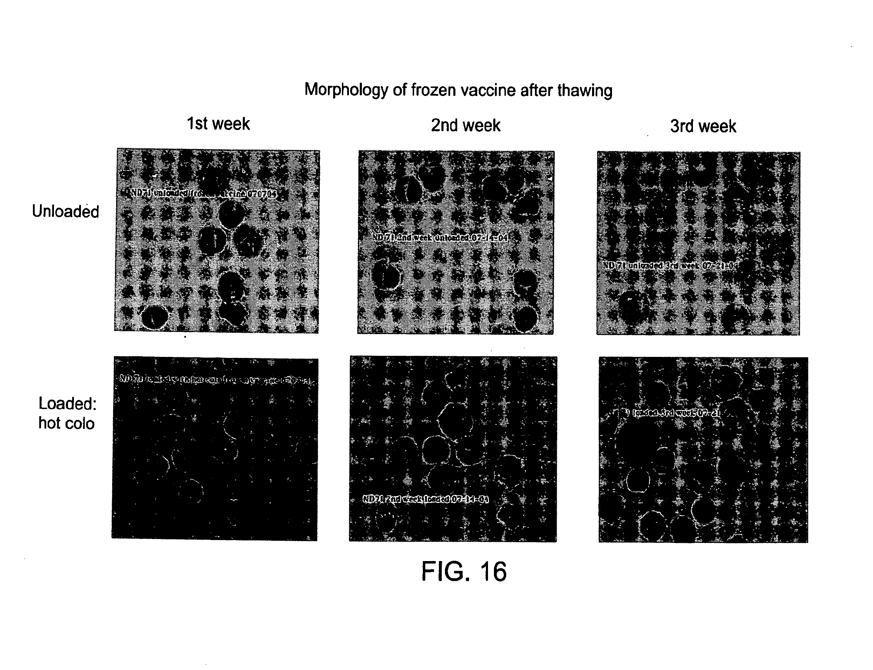

Figure 15 shows detailed steps and a timeline for the manufacture of the vaccine of the present invention in which the cells are provided in an elutriation bag (step 26) and transferred to one or more culture bags (step 28). In step 30, for example, over three culture days one or more cytokines are provided at hour zero. Following the exposure of the cells to cytokines the cells are exposed to killed target cells, e.g., Colo629 cells and optionally additional cytokines. Finally, the cells may be harvested after about 72 and/or frozen, tested, sterilized and the like. IFNα-DCs were generated in culture bags either unloaded or loaded with killed Colo829 cells, frozen and stored at -80°C for 1, 2 and 3 weeks. Frozen cells were thawed at weeks 1, 2 and 3 and their morphology was assessed by Giemsa staining. As shown in Figure 16, both loaded and unloaded IFN-DCs retained DC morphology after freezing/thawing.

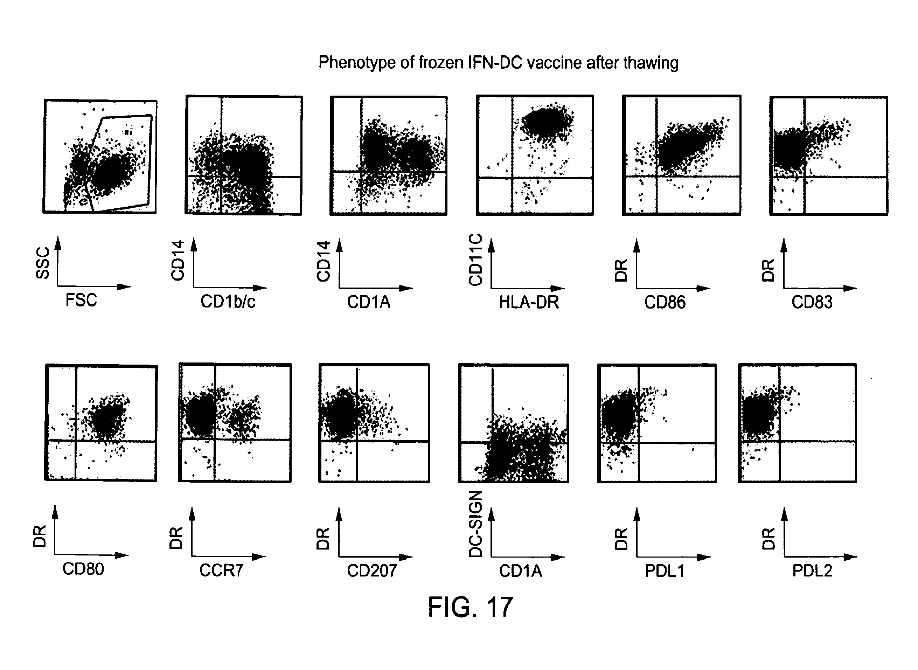

Figure 17 shows an example of frozen/thawed vaccine phenotype as analyzed by surface staining with indicated antibodies and flow cytometry. The DCs show expected phenotype consistent with their generation in the presence of IFN-alpha including: expression of CD1 molecules (CD1a and CDlb/c), expression of CD14 consistent with IFN-DCs being interstitial DCs; high level of HLA-DR and co-stimulatory CD80 and CD86 molecules. Thus, frozen/thawed vaccines retain the morphology and phenotype of IFN-DCs.

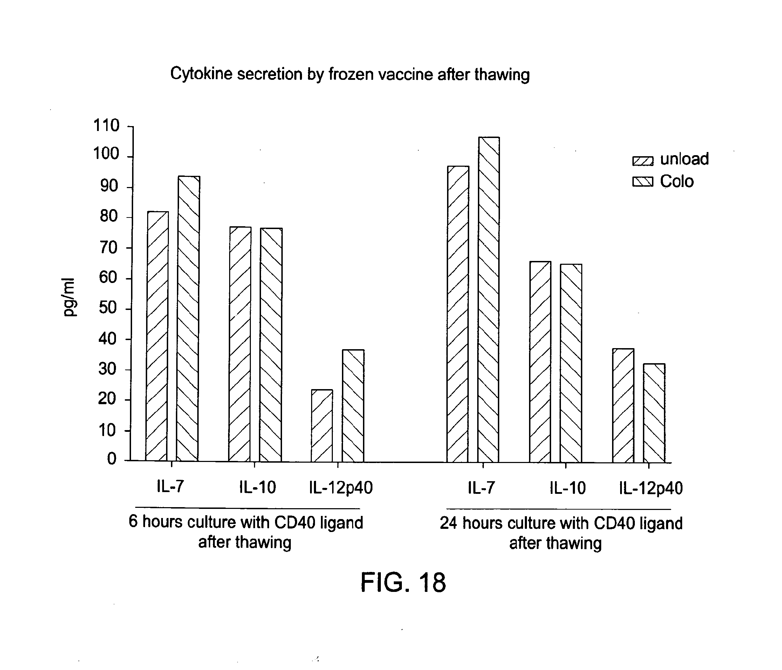

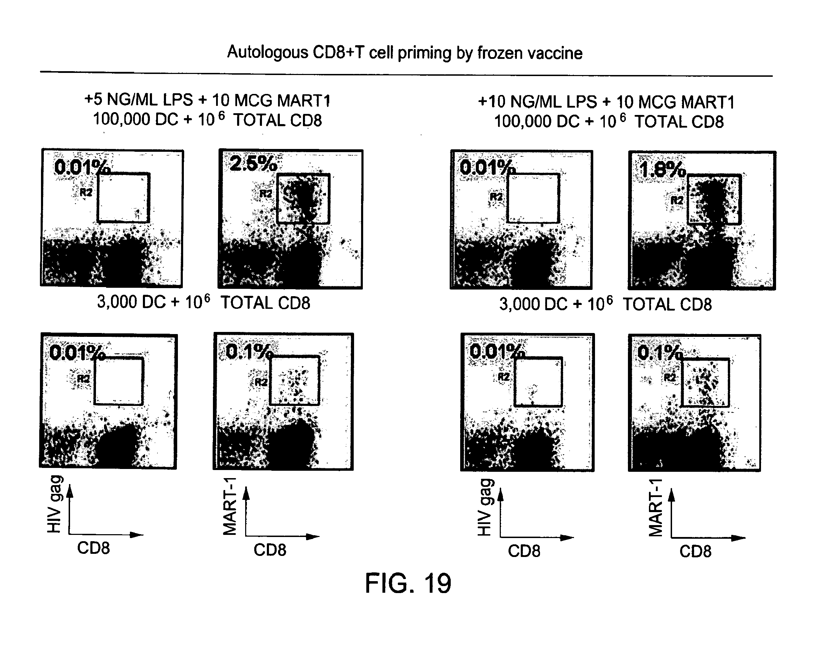

Upon interaction with T cells, DCs secrete cytokines that regulate T cell differentiation. Therefore, we assessed cytokines secreted by frozen/thawed IFN-DCs (either unloaded or loaded with killed Colo829 cells) when exposed to soluble CD40 ligand to replace T cell signal. Supernatants were assessed after 6 and 24 hrs culture using Multiplex Cytokine Analysis (Luminex). The three major cytokines secreted at levels >1ng/ml included IL-8 (∼10ng/ml), IL-6 and MIP1 alpha. As expected from our pre-clinical studies, IFN-DCs secreted IL-7 (Figure 19). Furthermore, low levels of IL-10 could be detected (<100 pg/ml). However, IL-10 secretion was not due to loading with killed Colo829 cells as the levels were similar in cultures of unloaded or loaded DCs (Figure 18). Finally, IL-10 secretion appeared donor-related (data not shown).

The ultimate parameter of a DC vaccine, is its capacity to present tumor antigen to autologous CD8+T cells and induce their differentiation. Thus, frozen/thawed HLA-A*0201+IFN-DCs were stimulated for 24hrs with LPS (5 or 10 ng/ml), pulsed in the last 10hrs with MART-1 peptide and used as stimulators of purified autologous CD8⁺T cells. T cell cultures were boosted once at day 7, supplemented with IL-7 and IL-2 and T cell differentiation was assessed by tetramer staining at day 5 after boost. As shown in Figure 19, at the DC:T cell ratio 1:10 (i.e., 10⁵ DCs per 10⁶ T cells) at day 12 of culture, ∼ 2% of CD8+T cells specifically bound MART-1 tetramer. Furthermore, the differentiation of MART-1 specific CD8+T cells could observed even at the DC:T cell ratio as low as 1:33, i.e., 3000 DCs per 10⁶ T cells. These results demonstrate that frozen IFN-DCs retain their morphology, phenotype and capacity to expand antigen-specific CD8+T cells.

Preliminary data on the feasibility of product manufacture and release in patients with metastatic melanoma. To date we have gathered data on product manufacture in 7 patients with stage IV melanoma. These are preliminary results obtained in the course of an ongoing clinical trial (IRB#005-065, IND#12339). These preliminary lot manufacture data using cells from patients with metastatic melanoma who underwent chemotherapy suggest feasibility of the process which was developed using cells from healthy donors as described in prior sections. Subsequent tables summarize lots manufactured to date using patient's material.

**Table 7: Patients with stage IV melanoma: Monocyte recovery from Elutra fraction 5**

| **Pt** | **Cell Concentration X 10 (6)** | **% CD14+** |
|---|---|---|
| 005-065-02-001* | 58 | 87 |
| 005-065-02-002 | 2150 | 88 |
| 005-065-02-004 | 4150 | 53 |
| 005-065-02-005 | 2850 | 73 |
| 005-065-02-006 | 2000 | 77 |
| 005-065-02-007 | 1600 | 88 |
| 005-065-02-008 | 3650 | 80 |
| **Average** | **2351** | **78%** |
| **SD** | **1364** | **13%** |

| | | |
|---|---|---|
| * Due to poor quality of the apheresis product this patient showed monocytes in all fractions post-elutriation. To insure sufficient number of vaccines all fractions were pooled for culture. | | |

**Table 8: Patients with stage IV melanoma: DC recovery in lots produced to date**

| **Pt** | **No. Bags** | **No. Cells Cultured X 10(6)** | **No. Cells Recovered x 10(6)** | **% Recovery** | **% Viability prior to freezing** | **No. DC x 10(6) / frozen vial** | **No. Vials** |
|---|---|---|---|---|---|---|---|
| 005-065-02-001* | 2 | 199 | 75 | 38 | 81 | 20 | 7 |
| | 3 | F4&5 256 | F4&5 67 | 26 | 69 | 10 | 1 |
| 005-065-02-002 | 10 | 1000 | 537 | 54 | 90 | 30 | 18 |
| 005-065-02-004 | 17 | 1700 | 788 | 46 | 91 | 32 | 21 |
| 005-065-02-005 | 12 | 1200 | 624 | 52 | 94 | 31 | 20 |
| 005-065-02-006 | 10 | 1000 | 564 | 56 | 94 | 27 | 19 |
| 005-065-02-007 | 16 | 1600 | 917 | 57 | 98 | 31 | 30 |
| 005-065-02-008 | 16 | 1600 | 928 | 58 | 85 | 35 | 28 |
| **Average** | | **1350** | **726** | **54** | **92** | | |
| **SD** | | **321** | **175** | **4** | **5** | | |

**Table 9: Patients with stage IV melanoma: DC viability post-thaw without DMSO wash out**

| **Patient Number** | **Vial Number** | **Date of Manufacture** | **%Viability post thaw** |
|---|---|---|---|
| 005-065-02-001 | 8 | 09/30/05 | 66 |
| 005-065-02-003 | 18 | 10/02/05 | 65 |
| 005-065-02-003 | 1 | 10/02/05 | 63 |
| 005-065-02-004 | 1 | 10/09/05 | 79 |
| 005-065-02-004 | 10 | 10/09/05 | 76 |
| 005-065-02-004 | 21 | 10/09/05 | 77 |
| 005-065-02-005 | 1 | 10/20/05 | 63 |
| 005-065-02-005 | 10 | 10/20/05 | 65 |
| 005-065-02-005 | 20 | 10/20/05 | 70 |
| 005-065-02-006 | 1 | 10/27/05 | 50 |
| 005-065-02-006 | 9 | 10/27/05 | 56 |
| 005-065-02-006 | 19 | 10/27/05 | 47 |
| 005-065-02-007 | 1 | 11/20/05 | 90 |
| 005-065-02-007 | 18 | 11/20/05 | 91 |
| 005-065-02-007 | 30 | 11/20/05 | 88 |
| 005-065-02-008 | 1 | 12/21/05 | 67 |
| 005-065-02-008 | 14 | 12/21/05 | 72 |
| **Average** | | | **70** |
| **SD** | | | **13** |

**Table 10: Patients with stage IV melanoma: DC recovery post-thaw without DMSO wash out**

| **Patient** | **Vial** | **% Recovery** |
|---|---|---|
| 005-065-02-001 | 1 | 100 |
| 005-065-02-003 | 18 | 77 |
| 005-065-02-003 | 1 | 97 |
| 005-065-02-004 | 1 | 100 |
| 005-065-02-004 | 10 | 100 |
| 005-065-02-004 | 21 | 100 |
| 005-065-02-005 | 1 | 68 |
| 005-065-02-005 | 10 | 93 |
| 005-065-02-005 | 20 | 90 |
| 005-065-02-006 | 1 | 47 |
| 005-065-02-006 | 9 | 63 |
| 005-065-02-006 | 19 | 69 |
| 005-065-02-007 | 1 | 100 |
| 005-065-02-007 | 18 | 100 |
| 005-065-02-007 | 30 | 100 |
| 005-065-02-008 | 1 | 57 |
| 005-065-02-008 | 14 | 56 |
| 005-065-02-008 | 26 | 72 |
| **Average** | | **83** |
| **SD** | | **19** |

Preferably the present invention is defined as follows:
Definition 1. A method of making a composition for treating cancer comprising the steps of:
   activating one or more antigen presenting cells that present one or more cancer antigens
   and induce T cell activation through incubation of the one or more antigen presenting cells activated with GM-CSF and interferon alpha in the presence of one or more cancer cells that are heat shocked and subsequently killed.
Definition 2. The method of definition 1, further comprising the step of pulsing the antigen presenting cells with an antigen comprising the remains of one or more cancer cells that are heat shocked and subsequently killed under conditions that induce T-cell activation.
Definition 3. The method of definition 1, further comprising the step of storing cryogenically the one or more antigen presenting cells that have been pulsed with the cancer cells that are heat shocked and subsequently killed.
Definition 4. The method of definition 1, wherein the one or more antigen presenting cells comprise antigen presenting dendritic cells that present one or more cancer specific antigen obtained after heat shock and killing of the cancer cells to one or more CDS+ T cells.
Definition 5. The method of definition 1, wherein the antigen presenting cells are dendritic cells, monocytes, autologous cells, heterologous cells or a combination thereof.
Definition 6. The method of definition 1, wherein the one or more antigen presenting dendritic cells comprise GM-CSF and IFNα-induced dendritic cells.
Definition 7. The method of definition 1, wherein the one or more antigen presenting cells comprise monocytes are cultured with GM-CSF and IFNα.
Definition 8. The method of definition 1, wherein the one or more cancer cells are defined further as heat, inactivated melanoma cells derived one or more Colo829 melanoma cells.
Definition 9. The method of definition 1, wherein the one or more cancer cells are defined further as heat inactivated melanoma cells are the same cancer cell type as the patient.
Definition 10. The method of definition 1, wherein the cancer cells comprise from a patient.
Definition 11. The method of claim 1, wherein the cancer cells comprise an established cancer cell lines.
Definition 12. The method of claim 1, wherein the cancer cells are melanoma cells.
Definition 13. The method of claim 1, wherein the one or more cancer cells are defined further as melanoma cells that comprise one or more Mel-2 cells, one or more Mel-3 cells, one or more Mel-4 cells, one or more Mel-6 cells, one or more Mel-9 cells or a combination thereof.
Definition 14. The method of definition 1, wherein the cancer cells are treated by heating for about 4 hours at between about 38°C and about 46°C.
Definition 15. The method of definition 1, wherein the cancer cells are killed by γ-irradiation for about 0.5 hours at about 160Gy.
Definition 16. The method of definition 1, wherein the cancer cells are killed by the heating.
Definition 17. The method of definition 1, further comprising the addition of one or more pulsed antigen presenting cells comprising enucleated dendritic cells and one or more heat treated cancer cells capable of inducing T cell activation, pulsed with a preparation.
Definition 19. The method of definition 17, wherein the cancer cells are selected from the group consisting of fibrosarcoma, myxosarcoma, liposarcoma, chondrosarcoma, osteogenic sarcoma, chordoma, angiosarcoma, Kaposi's sarcoma, endotheliosarcoma, lymphangiosarcoma, lymphangioendotheliosarcoma, synovioma, mesothelioma, Ewing's tumor, leiomyosarcoma, rhabdomyosarcoma, rhabdosarcoma, colorectal carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duet carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, neuroblastoma, retinoblastoma, myeloma, lymphoma, and leukemia. 18. The method of claim 1, wherein the cancer cells are from cancers selected from the group consisting of fibrosarcoma, myxosarcoma, Liposarcoma, chondrosarcoma, osteogenic sarcoma, chordoma, angiosarcoma, Kaposi's sarcoma, endotheliosarcoma, lymphangiosarcoma, lymphangioendotheliosarcoma, synovioma, mesothelioma, Ewing's tumor, leiomyosarcoma, rhabdomyosarcoma, rhabdosarcoma, colorectal carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma acoustic neuroma, oligodendroglioma, meningioma, neuroblastoma, retinoblastoma, myeloma, lymphoma, and leukemia.
Definition 20. A method of making a dendritic cell that presents cancer antigens comprising the steps of:
   differentiating one or more monocytes into one or more dendritic cells; and loading the one or more dendritic cells with an antigen presenting composition comprising one or more cancer cells that are heat shocked and subsequently killed, wherein the one or more dendritic cells are incubated with GM-CSF and IFNα under conditions that cause the one or more dendritic cells to present one or more antigens in the composition.
Definition 21. A method of making an antigen presenting dendritic cell that presents melanoma antigens comprising the steps of:
   isolating one or more monocytes from a patient suspected of having cancer; maturing the one or more monocytes be exposure of the monocytes to GM-CSF and IFNα in cell culture into one or more matured dendritic cells; and
   loading the one or more matured dendritic cells with an antigens presenting composition comprising one or more cancer cells that are heat shocked and subsequently killed, wherein the loaded one or more matured dendritic cells are capable of inducing T cell activation and under conditions that allow the one or more matured dendritic cells to present one or more antigens in the composition.
Definition 22. A cancer-specific vaccine capable of inducing T cell activation comprising:
   a pharmaceutically effective amount of one or more dendritic cells activated with GM-CSF and IFNα to present antigen and which dendritic cells present one or more antigens that comprise one or more cancer cells that have been heat shocked and killed after the heat shock. Definition 23. The vaccine of definition 22, wherein the cancer cells are not apoptotic at the time of killing.
Definition 24. A melanoma specific vaccine capable of inducing T cell activation comprising:
   a pharmaceutically effective amount of one or more GM-CSF and IFNα-induced dendritic cells loaded and presenting as an antigen, heat shocked and killed melanoma cells that are not apoptotic, wherein the one or more IFNα dendritic cells are incubated under conditions that promote the presentation of the one or more melanoma.
Definition 25. The vaccine of definition 24, wherein the pulsed preparation for T-cell activation is further defined as comprising one or more pulsed enucleated antigen presenting cells.
Definition 26. The vaccine of definition 24, wherein the vaccine is cryogenically preserved.
Definition 27. The vaccine of definition 24, wherein the one or more antigen presenting dendritic cells presents an autologous cancer antigen to one or more CD8+ T cells to trigger differentiation.
Definition 28. The vaccine of definition 24, wherein the antigen presenting cells are dendritic cells, monocytes, autologous cells, heterologous cells or a combination thereof.
Definition 29. The vaccine of definition 24, wherein the one or more monocytes are cultured with GM-CSF and IFNα.
Definition 30. The vaccine of definition 24, wherein the one or more heat inactivated melanoma cells are derived from the patient.
Definition 31. The vaccine of definition 24, wherein the cancer cells are treated by heating for about 4 hours at between about 38°C and about 46°C.
Definition 32. The vaccine of definition 24, wherein the cancer cells are killed by γ-irradiation for about 0.5 hours at about 160Gy.
Definition 33. The vaccine of definition 24, wherein the cancer cells are from cancers selected from the group consisting of fibrosarcoma, myxosarcoma, Liposarcoma, chondrosarcoma, osteogenic sarcoma, chordoma, angiosarcoma, Kaposi's sarcoma, endotheliosarcoma, lymphangiosarcoma, lymphangioendotheliosarcoma, synovioma, mesothelioma, Swing's tumor, leiomyosarcoma, rhabdomyosarcoma, rhabdosarcoma, colorectal carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, neuroblastoma, retinoblastoma, myeloma, lymphoma, and leukemia.
Definition 34. The method of definition 25, wherein the cancer cells are selected from the group consisting of fibrosarcoma, myxosarcoma, Liposarcotna, chondrosarcoma, osteogenic sarcoma, chordoma, angiosarcoma, Kaposi's sarcoma, endotheliosarcoma, lymphangiosarcoma, lymphangioendotheliosarcoma, synovioma, mesothelioma, Ewing's tumor, leiomyosarcoma, rhabdomyosarcoma, rhabdosarcoma, colorectat carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, glioma, astrocytorna, medulloblastoma, craniopharyngioma, ependymoma, pinealoma, hemangioblastoma, acoustic neuroma, oligodendroglioma, meningioma, neuroblastoma, retinoblastoma, myeloma, lymphoma, and leukemia.
Definition 35. A method of treating a patient suspected of having cancerous cell growth comprising the steps of:
   loading one or more cancer antigens on dendritic cell activated with GM-CSF and IFNα under conditions that induce antigen presentation and T-cell activation, wherein the cancer antigens comprise cancer cells that have been heat shocked and subsequently killed; and
   administering to the patient in need thereof the one or more dendritic cells that present one or more cancer antigens to activate T cells.
Definition 36. A method of treated a patient suspected of having cancer comprising the steps of:
   isolating and maturing one or more monocytes exposed to GM-CSF and IFNα;
   loading the one or more dendritic cells capable of inducing T cell activation with a composition comprising one or more heat treated cancer cells that are heat shocked and subsequently killed to form one or more antigen presenting dendritic cells; and
   administering the one or more antigen presenting dendritic cells to the patient in need thereof.

### INVENTOR'S REFERENCES

Banchereau, J., Palucka, A.K., Dhodapkar, M., Burkeholder, S., Taquet, N., Alexandre, R., Taquet, S., Coquery, S., Wittkowski, K., Bhardwaj, N., Pineiro, L., Steinman, R., and Fay, J. (2001). Immune and clinical responses in patients with metastatic melanoma to CD34+ progenitor-derived dendritic cell vaccine. Cancer Research. 61:6451-6458.
Blanco, P., Palucka, A.K., Gill, M., Pascual, V., and Banchereau, J. (2001). Interferon alpha and dendritic cells in SLE. Science. 294:1540-1543.
Mohamadzadeh, M., Berard, F., Essert, G., Chalouni, C., Pulendran, B., Davoust, J., Bridges, G., Palucka, A.K., and Banchereau, J. (2001). Interleukin 15 skews monocyte differentiation into dendritic cells with features of Langerhans cells. J.Exp.Med. 194:1013-20.
Chomarat, P., Dantin, C., Bennett, L., Banchereau, J., and Palucka, A.K. (2003) TNF skews monocyte differentiation from macrophages to dendritic cells. J. Immunol. 171:2262-9
Palucka, A.K., Dhodapkar, M., Burkeholder, S., Wittkowski, K., Steinman, R., Fay, J., and Banchereau, J. (2003). CD34+ progenitor-derived dendritic cell vaccine permits rapid induction of T cell immunity in patients with metastatic melanoma J. Immunotherapy. 26(5):432-9.
Neihardt-Berard, E-M., Berard, F., Banchereau, J. and Palucka, A.K. (2004) Dendritic cells loaded with killed breast cancer cells induce differentiation of tumor-specific cytotoxic T lymphocytes. Breast Cancer Res 2004, 6:R322-R328.
Paczesny, S., Banchereau, J., Fay, J., and Palucka, A.K. (2004) Expansion of melanoma-specific cytolytic CD8+ T cell precursors in patients with metastatic melanoma vaccinated with CD34+ progenitor-derived dendritic cells. J Exp Med 199(11):1503-11
Paczesny, S., Shi, H., Saito, H., Mannoni, P., Fay, J., Banchereau, J and Palucka, AK. (2005) Measuring Melanoma-specific CTLs Elicited by Dendritic Cell Vaccines with a Tumor Inhibition Assay in vitro. J. Immunotherapy 28(2):148-157
Palucka, AK., Dhodapkar, M., Paczesny, S., Ueno, H., Fay, J., and Banchereau, J. (2005) Boosting vaccinations with peptide-pulsed CD34+ progenitor derived dendritic cells can expand long-lived melanoma-specific CD8+ T cell immunity in patients with metastatic melanoma. J. Immunotherapy 28(2):158-168
Shi, H., Palucka, AK. Chapel, S., Bagnis, C., Mannoni, P., Davoust, J., and Banchereau, J. (2005) Enhanced cross-priming of melanoma-specific CTLs by dendritic cells loaded with killed allogeneic melanoma cells that were treated with hyperthermia. J Immunol (in revision)
Fay, J., Palucka, A.K., Paczesny, S., Ueno, H., Dhodapkar, M., Burkeholder, S., Steinman, R., and Banchereau, J. (2005) Long-term outcomes in patients with metastatic melanoma vaccinated with peptide-pulsed CD34-DCs. Cancer Immunol Immunother
Banchereau, J., Ueno, H., Dhodapkar, M., Connolly, J.E., Finholt-Perry, J., Klechevsky, E., Blanck, J-P., Johnston, DA., Steinman, R., Palucka, AK., Fay, J. (2005) Immune and clinical outcomes in patients with stage IV melanoma vaccinated with peptide-pulsed dendritic cells derived from CD34+ progenitors and activated with type I interferon. J Immunother.
Dubsky, P., Saito, H., Dantin, C., Connolly, J., Banchereau, J. and Palucka, AK. (2005) IL-15-induced human dendritic cells efficiently prime low frequency melanoma-specific naïve CD8+T cells to differentiate into cytotoxic T cells. Submitted
Saito, H., Dubsky, P., Dantin, C., Finn, OJ., Banchereau, J. and Palucka, AK. Cross-priming of Cyclin B1, MUC-1 and survivin peptide-specific CD8+T cells by dendritic cells loaded with killed allogeneic breast cancer cells. Submitted.
Ueno, H., Connolly, JE., Vence, L., Palucka, AK., and Banchereau, J. (2005) Global assessment of tumor-antigen specific human T cell repertoire. In preparation
Palucka, AK., Ueno, H., Connolly, J., Kerneis-Norvell, F., Blanck, J-P., Johnston, DA., Fay, J., and Banchereau, J. (2005) Immune and clinical responses in patients with stage IV melanoma vaccinated with dendritic cells loaded with killed allogeneic melanoma cells. In revision

### OTHER REFERENCES:

1. Steinman, R. M. The dendritic cell system and its role in immunogenicity. Annu Rev Immunol 9, 271-96 (1991).
2. Banchereau, J. et al. Immunobiology of dendritic cells. Ann Rev Immunol 18, 767-811 (2000).
3. Mellman, I. & Steinman, R. M. Dendritic cells: specialized and regulated antigen processing machines. Cell 106, 255-8 (2001).
4. Caux, C. et al. CD34+ hematopoietic progenitors from human cord blood differentiate along two independent dendritic cell pathways in response to granulocyte-macrophage colony-stimulating factor plus tumor necrosis factor alpha: II. Functional analysis. Blood 90, 1458-70 (1997).
5. Jego, G. et al. Plasmacytoid dendritic cells induce plasma cell differentiation through type I interferon and interleukin 6. Immunity 19, 225-34 (2003).
6. Fernandez, N. C. et al. Dendritic cells directly trigger NK cell functions: cross-talk relevant in innate anti-tumor immune responses in vivo. Nat Med 5, 405-11 (1999).
7. Kadowaki, N. et al. Distinct cytokine profiles of neonatal natural killer T cells after expansion with subsets of dendritic cells. JExp Med 193, 1221-6 (2001).
8. Davis, I. D., Jefford, M., Parente, P. & Cebon, J. Rational approaches to human cancer immunotherapy. JLeukoc Biol 73, 3-29 (2003).
9. Gilboa, E., Nair, S. K. & Lyerly, H. K. Immunotherapy of cancer with dendritic-cell-based vaccines. Cancer Immunol Immunother 46, 82-7 (1998).
10. Lu, W., Arraes, L. C., Ferreira, W. T. & Andrieu, J. M. Therapeutic dendritic-cell vaccine for chronic HIV-1 infection. Nat Med 10, 1359-1365 (2004).
11. Townsend, A. R., Gotch, F. M. & Davey, J. Cytotoxic T cells recognize fragments of the influenza nucleoprotein. Cell 42, 457-67 (1985).
12. Boon, T., Cerottini, J. C., Van den Eynde, B., van der Bruggen, P. & Van Pel, A. Tumor antigens recognized by T lymphocytes. Annu Rev Immunol 12, 337-65 (1994).
13. Rosenberg, S. A. Cancer vaccines based on the identification of genes encoding cancer regression antigens. Immunol Today 18, 175-82 (1997).
14. Pardoll, D. M. Cancer vaccines. Nat Med 4, 525-31 (1998).
15. Gilboa, E. The makings of a tumor rejection antigen. Immunity 11, 263-70 (1999).
16. Finn, O. J. Cancer vaccines: between the idea and the reality. Nat Rev Immunol 3, 630-41 (2003).
17. Antonia, S., Mule, J. J. & Weber, J. S. Current developments of immunotherapy in the clinic. Curr Opin Immunol 16, 130-6 (2004).
18. Hsueh, E. C. & Morton, D. L. Antigen-based immunotherapy of melanoma: Canvaxin therapeutic polyvalent cancer vaccine. Semin Cancer Biol 13, 401-7 (2003).
19. Sondak, V. K. & Sosman, J. A. Results of clinical trials with an allogenic melanoma tumor cell lysate vaccine: Melacine. Semin Cancer Biol 13, 409-15 (2003).
20. Steinman, R. M., Hawiger, D. & Nussenzweig, M. C. Tolerogenic dendritic cells. Annu Rev Immunol 21, 685-711 (2003).
21. Caux, C., Dezutter-Dambuyant, C., Schmitt, D. & Banchereau, J. GM-CSF and TNF-alpha cooperate in the generation of dendritic Langerhans cells. Nature 360, 258-61 (1992).
22. Romani, N. et al. Proliferating dendritic cell progenitors in human blood. J Exp Med 180, 83-93 (1994).
23. Sallusto, F. & Lanzavecchia, A. Efficient presentation of soluble antigen by cultured human dendritic cells is maintained by granulocyte/macrophage colony-stimulating factor plus interleukin 4 and downregulated by tumor necrosis factor alpha. J Exp Med 179, 1109-18 (1994).
24. Peters, J. H. et al. Signals required for differentiating dendritic cells from human monocytes in vitro. Adv Exp Med Biol 329, 275-80 (1993).
25. Nestle, F. O. et al. Vaccination of melanoma patients with peptide- or tumor lysate-pulsed dendritic cells. Nat Med 4, 328-32 (1998).
26. Thurner, B. et al. Vaccination with mage-3A1 peptide-pulsed mature, monocyte-derived dendritic cells expands specific cytotoxic T cells and induces regression of some metastases in advanced stage IV melanoma. J Exp Med 190, 1669-78 (1999).
27. Mohamadzadeh, M. et al. Interleukin 15 skews monocyte differentiation into dendritic cells with features of Langerhans cells. J Exp Med 194, 1013-20 (2001).
28. Santini, S. M. et al. Type I interferon as a powerful adjuvant for monocyte-derived dendritic cell development and activity in vitro and in Hu-PBL-SCID mice. J Exp Med 191, 1777-88. (2000).
29. Wang, R. F., Wang, X., Atwood, A. C., Topalian, S. L. & Rosenberg, S. A. Cloning genes encoding MHC class II-restricted antigens: mutated CDC27 as a tumor antigen. Science 284, 1351-4 (1999).
30. Zitvogel, L. et al. Eradication of established murine tumors using a novel cell-free vaccine: dendritic cell-derived exosomes. Nat Med 4, 594-600 (1998).
31. Ribas, A., Butterfield, L. H., Glaspy, J. A. & Economou, J. S. Cancer immunotherapy using gene-modified dendritic cells. Curr Gene Ther 2, 57-78 (2002).
32. Boczkowski, D., Nair, S. K., Snyder, D. & Gilboa, E. Dendritic cells pulsed with RNA are potent antigen-presenting cells in vitro and in vivo. J Exp Med 184, 465-72 (1996).
33. Regnault, A. et al. Fcgamma receptor-mediated induction of dendritic cell maturation and major histocompatibility complex class I-restricted antigen presentation after immune complex internalization. J Exp Med 189, 371-80 (1999).
34. Fong, L. & Engleman, E. G. Dendritic cells in cancer immunotherapy. Annu Rev Immunol 18, 245-73 (2000).
35. Albert, M. L., Sauter, B. & Bhardwaj, N. Dendritic cells acquire antigen from apoptotic cells and induce class I- restricted CTLs. Nature 392, 86-9 (1998).
36. Berard, F. et al. Cross-Priming of Naive CD8 T Cells against Melanoma Antigens Using Dendritic Cells Loaded with Killed Allogeneic Melanoma Cells. J Exp Med 192, 1535-1544 (2000).
37. Neidhardt-Berard, E. M., Berard, F., Banchereau, J. & Palucka, A. K. Dendritic cells loaded with killed breast cancer cells induce differentiation of tumor-specific cytotoxic T lymphocytes. Breast Cancer Res 6, R322-8 (2004).
38. Apostolou, I., Sarukhan, A., Klein, L. & von Boehmer, H. Origin of regulatory T cells with known specificity for antigen. Nat Immunol 3, 756-63 (2002).
39. Jordan, M. S. et al. Thymic selection of CD4+CD25+ regulatory T cells induced by an agonist self-peptide. Nat Immunol 2, 301-6 (2001).
40. Bensinger, S. J., Bandeira, A., Jordan, M. S., Caton, A. J. & Laufer, T. M. Major histocompatibility complex class II-positive cortical epithelium mediates the selection of CD4(+)25(+) immunoregulatory T cells. J Exp Med 194, 427-38 (2001).
41. Fontenot, J. D., Gavin, M. A. & Rudensky, A. Y. Foxp3 programs the development and function of CD4+CD25+ regulatory T cells. Nat Immunol 4, 330-6 (2003).
42. Hori, S., Nomura, T. & Sakaguchi, S. Control of regulatory T cell development by the transcription factor Foxp3. Science 299, 1057-61 (2003).
43. Steitz, J., Bruck, J., Lenz, J., Knop, J. & Tuting, T. Depletion of CD25(+) CD4(+) T cells and treatment with tyrosinase-related protein 2-transduced dendritic cells enhance the interferon alpha-induced, CD8(+) T-cell-dependent immune defense of B16 melanoma. Cancer Res 61, 8643-6 (2001).
44. Sutmuller, R. P. et al. Synergism of cytotoxic T lymphocyte-associated antigen 4 blockade and depletion of CD25(+) regulatory T cells in antitumor therapy reveals alternative pathways for suppression of autoreactive cytotoxic T lymphocyte responses. J Exp Med 194, 823-32 (2001).
45. Golgher, D., Jones, E., Powrie, F., Elliott, T. & Gallimore, A. Depletion of CD25+ regulatory cells uncovers immune responses to shared murine tumor rejection antigens. Eur J Immunol 32, 3267-75 (2002).
46. Ghiringhelli, F. et al. CD4+CD25+ regulatory T cells suppress tumor immunity but are sensitive to cyclophosphamide which allows immunotherapy of established tumors to be curative. Eur J Immunol 34, 336-44 (2004).
47. Somasundaram, R. et al. Inhibition of cytolytic T lymphocyte proliferation by autologous CD4+/CD25+ regulatory T cells in a colorectal carcinoma patient is mediated by transforming growth factor-beta. Cancer Res 62, 5267-72 (2002).
48. Camara, N. O., Sebille, F. & Lechler, R. I. Human CD4+CD25+ regulatory cells have marked and sustained effects on CD8+ T cell activation. Eur J Immunol 33, 3473-83 (2003).
49. Azuma, T., Takahashi, T., Kunisato, A., Kitamura, T. & Hirai, H. Human CD4+ CD25+ regulatory T cells suppress NKT cell functions. Cancer Res 63, 4516-20 (2003).
50. Wolf, A. M. et al. Increase of regulatory T cells in the peripheral blood of cancer patients. Clin Cancer Res 9, 606-12 (2003).
51. Viguier, M. et al. Foxp3 expressing CD4+CD25(high) regulatory T cells are overrepresented in human metastatic melanoma lymph nodes and inhibit the function of infiltrating T cells. J Immunol 173, 1444-53 (2004).
52. Woo, E. Y. et al. Regulatory CD4(+)CD25(+) T cells in tumors from patients with early-stage non-small cell lung cancer and late-stage ovarian cancer. Cancer Res 61, 4766-72 (2001).
53. Curiel, T. J. et al. Specific recruitment of regulatory T cells in ovarian carcinoma fosters immune privilege and predicts reduced survival. Nat Med 10, 942-9 (2004).
54. Wang, H. Y. et al. Tumor-specific human CD4+ regulatory T cells and their ligands: implications for immunotherapy. Immunity 20, 107-18 (2004).
55. Woo, E. Y. et al. Cutting edge: Regulatory T cells from lung cancer patients directly inhibit autologous T cell proliferation. J Immunol 168, 4272-6 (2002).
56. Dieckmann, D., Bruett, C. H., Ploettner, H., Lutz, M. B. & Schuler, G. Human CD4(+)CD25(+) regulatory, contact-dependent T cells induce interleukin 10-producing, contact-independent type 1-like regulatory T cells [corrected]. J Exp Med 196, 247-53 (2002).
57. Jonuleit, H. et al. Infectious tolerance: human CD25(+) regulatory T cells convey suppressor activity to conventional CD4(+) T helper cells. J Exp Med 196, 255-60 (2002).
58. Roncarolo, M. G., Bacchetta, R., Bordignon, C., Narula, S. & Levings, M. K. Type 1 T regulatory cells. Immunol Rev 182, 68-79 (2001).
59. Groux, H. et al. A CD4+ T-cell subset inhibits antigen-specific T-cell responses and prevents colitis. Nature 389, 737-42 (1997).
60. Chen, Y., Kuchroo, V. K., Inobe, J., Hafler, D. A. & Weiner, H. L. Regulatory T cell clones induced by oral tolerance: suppression of autoimmune encephalomyelitis. Science 265, 1237-40 (1994).
61. Jonuleit, H., Schmitt, E., Schuler, G., Knop, J. & Enk, A. H. Induction of interleukin 10-producing, nonproliferating CD4(+) T cells with regulatory properties by repetitive stimulation with allogeneic immature human dendritic cells. J Exp Med 192, 1213-22 (2000).
62. Wakkach, A. et al. Characterization of dendritic cells that induce tolerance and T regulatory 1 cell differentiation in vivo. Immunity 18, 605-17 (2003).
63. Monti, P. et al. Tumor-derived MUC1 mucins interact with differentiating monocytes and induce IL-10highIL-12low regulatory dendritic cell. J Immunol 172, 7341-9 (2004).
64. Dhodapkar, M. V. & Steinman, R. M. Antigen-bearing immature dendritic cells induce peptide-specific CD8(+) regulatory T cells in vivo in humans. Blood 100, 174-7 (2002).
65. North, R. J. The murine antitumor immune response and its therapeutic manipulation. Adv Immunol 35, 89-155 (1984).
66. Berd, D., Maguire, H. C., Jr. & Mastrangelo, M. J. Induction of cell-mediated immunity to autologous melanoma cells and regression of metastases after treatment with a melanoma cell vaccine preceded by cyclophosphamide. Cancer Res 46, 2572-7 (1986).
67. Berd, D., Maguire, H. C., Jr., McCue, P. & Mastrangelo, M. J. Treatment of metastatic melanoma with an autologous tumor-cell vaccine: clinical and immunologic results in 64 patients. J Clin Oncol 8, 1858-67 (1990).
68. Morton, D. L. et al. Polyvalent melanoma vaccine improves survival of patients with metastatic melanoma. Ann N Y Acad Sci 690, 120-34 (1993).
69. Mitchell, M. S. Perspective on allogeneic melanoma lysates in active specific immunotherapy. Semin Oncol 25, 623-35 (1998).
70. Bystryn, J. C. et al. Immunogenicity of a polyvalent melanoma antigen vaccine in humans. Cancer 61, 1065-70 (1988).
71. Livingston, P. O. et al. Vaccines containing purified GM2 ganglioside elicit GM2 antibodies in melanoma patients. Proc Natl Acad Sci U S A 84, 2911-5 (1987).
72. Hoon, D. S., Foshag, L. J., Nizze, A. S., Bohman, R. & Morton, D. L. Suppressor cell activity in a randomized trial of patients receiving active specific immunotherapy with melanoma cell vaccine and low dosages of cyclophosphamide. Cancer Res 50, 5358-64 (1990).
73. Holtl, L. et al. Allogeneic dendritic cell vaccination against metastatic renal cell carcinoma with or without cyclophosphamide. Cancer Immunol Immunother 54, 663-70 (2005).
74. Siegal, F. P. et al. The nature of the principal type 1 interferon-producing cells in human blood [In Process Citation]. Science 284, 1835-7 (1999).
75. Cella, M. et al. Maturation, activation, and protection of dendritic cells induced by double-stranded RNA. J Exp Med 189, 821-9 (1999).
76. Montoya, M. et al. Type I interferons produced by dendritic cells promote their phenotypic and functional activation. Blood 99, 3263-71 (2002).
77. Sprent, J., Tough, D. F. & Sun, S. Factors controlling the turnover of T memory cells. Immunol Rev 156, 79-85 (1997).
78. Marrack, P., Kappler, J. & Mitchell, T. Type I interferons keep activated T cells alive. J Exp Med 189, 521-30 (1999).
79. Le Bon, A. et al. Type i interferons potently enhance humoral immunity and can promote isotype switching by stimulating dendritic cells in vivo. Immunity 14, 461-70 (2001).
80. Proietti, E. et al. Type I IFN as a natural adjuvant for a protective immune response: lessons from the influenza vaccine model. J Immunol 169, 375-83 (2002).
81. Blanco, P., Palucka, A. K., Gill, M., Pascual, V. & Banchereau, J. Induction of dendritic cell differentiation by IFN-alpha in systemic lupus erythematosus. Science 294, 1540-3 (2001).
82. Bennett, L. et al. Interferon and granulopoiesis signatures in systemic lupus erythematosus blood. J Exp Med 197, 711-23 (2003).
83. Karaghiosoff, M. et al. Central role for type I interferons and Tyk2 in lipopolysaccharide-induced endotoxin shock. Nat Immunol 4, 471-7 (2003).
84. Woodhall, B., Pickrell, K. L., Georgiade, N. G., Mahaley, M. S. & Dukes, H. T. Effect of hyperthermia upon cancer chemotherapy; application to external cancers of head and face structures. Ann Surg 151, 750-9 (1960).
85. Mattson, D. et al. Heat shock and the activation of AP-1 and inhibition of NF-kappa B DNA-binding activity: possible role of intracellular redox status. Int J Hyperthermia 20, 224-33 (2004).
86. Gius, D., Mattson, D., Bradbury, C. M., Smart, D. K. & Spitz, D. R. Thermal stress and the disruption of redox-sensitive signalling and transcription factor activation: possible role in radiosensitization. Int J Hyperthermia 20, 213-23 (2004).
87. Berwin, B. & Nicchitta, C. V. To find the road traveled to tumor immunity: the trafficking itineraries of molecular chaperones in antigen-presenting cells. Traffic 2, 690-7 (2001).
88. Basu, S. & Srivastava, P. K. Heat shock proteins: the fountainhead of innate and adaptive immune responses. Cell Stress Chaperones 5, 443-51 (2000).
89. Frydman, J. Folding of newly translated proteins in vivo: the role of molecular chaperones. Annu Rev Biochem 70, 603-47 (2001).
90. Srivastava, P. K., Udono, H., Blachere, N. E. & Li, Z. Heat shock proteins transfer peptides during antigen processing and CTL priming. Immunogenetics 39, 93-8 (1994).
91. Srivastava, P. Interaction of heat shock proteins with peptides and antigen presenting cells: Chaperoning of the Innate and Adaptive Immune Responses. Annu Rev Immunol 20, 395-425 (2002).
92. Basu, S., Binder, R. J., Ramalingam, T. & Srivastava, P. K. CD91 is a common receptor for heat shock proteins gp96, hsp90, hsp70, and calreticulin. Immunity 14, 303-13 (2001).
93. Becker, T., Hartl, F. U. & Wieland, F. CD40, an extracellular receptor for binding and uptake of Hsp70-peptide complexes. J Cell Biol 158, 1277-85 (2002).
94. Delneste, Y. et al. Involvement of LOX-1 in dendritic cell-mediated antigen cross-presentation. Immunity 17, 353-62 (2002).
95. Asea, A. et al. Novel signal transduction pathway utilized by extracellular HSP70: role of toll-like receptor (TLR) 2 and TLR4. JBiol Chem 277, 15028-34 (2002).

## Claims

1. An in vitro method of making a cancer-specific vaccine comprising exposing IFN dendritic cells capable of expressing IL-7 to killed cancer cells that had been previously heat shocked.

2. The method of claim 1 further comprising exposing the IFN dendritic cells to GM-CSF.

3. The method of claim 1 or 2, wherein the dendritic cells present one or more specific antigen obtained after heat shock and killing of the cancer cells to one or more CD8+ T cells.

4. The method of any of claims 1 to 3, wherein the one or more cancer cells are melanoma cells.

5. The method of any one of claims 1 to 4, wherein the cancer cells comprise an established cancer cell line.

6. The method of any one of claims 1 to 5, wherein the cancer cells comprise one or more Colo829 melanoma cells.

7. The method of any one of claims 1 to 6, wherein the cancer cells are melanoma cells.

8. The method of any one of claims 1 to 7, wherein the one or more cancer cells are defined further as melanoma cells that comprise one or more Mel-2 cells, one or more Mel-3 cells, one or more Mel-4 cells, one or more Mel-6 cells, one or more Mel-9 cells or a combination thereof.

9. The method of any one of claims 1 to 8, wherein the cancer cells are from cancers selected from the group consisting of fibrosarcoma, myxosarcoma, liposarcoma, chondrosarcoma, osteogenic sarcoma, chordoma, angiosarcoma, Kaposi's sarcoma, endotheliosarcoma, lymphangiosarcoma, lymphangioendotheliosarcoma, synovioma, mesothelioma, Ewing's tumor, leiomyosarcoma, rhabdomyosarcoma, rhabdosarcoma, colorectal carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, neuroblastoma, retinoblastoma, myeloma, lymphoma, and leukemia.

10. The method of any one of claims 1 to 9, wherein the cancer cells are treated by heating for about 4 hours at between about 38°C and about 46°C.

11. The method of any one of claims 1 to 10, wherein the cancer cells are killed by γ-irradiation for about 0.5 hours at about 160Gy.

12. An in vitro method of making an antigen presenting dendritic cell that presents melanoma antigens comprising the steps of:
providing one or more monocytes which have been isolated from a patient suspected of having cancer; maturing the one or more monocytes by exposure of the monocytes to IFNα in cell culture into one or more IFN dendritic cells capable of expressing IL-7; and
loading the one or more IFN dendritic cells with an antigens presenting composition comprising one or more cancer cells that are heat shocked and subsequently killed, wherein the loaded one or more matured dendritic cells are capable of inducing T cell activation and under conditions that allow the one or more matured dendritic cells to present one or more antigens in the composition.

13. A cancer-specific vaccine capable of inducing T cell activation comprising:
a pharmaceutically effective amount of one or more dendritic cells derived from monocytes cultured with IFNα and activated with IFNα to express IL-7 and present antigens, the antigens **characterized in that** they comprise antigens of one or more cancer cells that have been heat shocked and killed after the heat shock.

14. The vaccine of claim 13, wherein the cancer cells are not apoptotic at the time of killing.

15. A melanoma specific vaccine capable of inducing T cell activation comprising:
a pharmaceutically effective amount of one or more dendritic cells derived from monocytes cultured with IFNα and expressing IL-7, loaded and presenting as an antigen, heat shocked and killed melanoma cells that are not apoptotic, wherein the one or more dendritic cells are incubated under conditions that promote the presentation of the one or more melanoma.

16. A cancer specific vaccine according to any of claims 13 to 15 for use in the treatment of cancer or cancerous cell growth in a patient.
